(19) Europäisches Patentamt
European Patent Office
Office européen des brevets

(11) Veröffentlichungsnummer : **0 071 216**
**B1**

(12) # EUROPÄISCHE PATENTSCHRIFT

(45) Veröffentlichungstag der Patentschrift : **30.12.86**

(21) Anmeldenummer : **82106690.9**

(22) Anmeldetag : **23.07.82**

(51) Int. Cl.[4] : **C 07 D207/273**, A 61 K 31/40, C 07 D403/12, C 07 D413/12, C 07 D405/12

(54) **3-Hydroxypyrrolidin-2-on-Derivate, Verfahren und Zwischenprodukte zu deren Herstellung und diese enthaltende Arzneimittel.**

(30) Priorität : **24.07.81 CH 4849/81**
**05.05.82 CH 2768/82**

(43) Veröffentlichungstag der Anmeldung : **09.02.83 Patentblatt 83/06**

(45) Bekanntmachung des Hinweises auf die Patenterteilung : **30.12.86 Patentblatt 86/52**

(84) Benannte Vertragsstaaten :
**AT BE CH DE FR IT LI LU NL SE**

(56) Entgegenhaltungen :
**EP-A- 0 024 030**
**DE-A- 2 759 033**
**GB-A- 1 309 692**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**

**CH-4002 Basel (CH)**

(72) Erfinder : **Aschwanden, Werner**
**Grellingerstrasse4**
**CH-4107 Ettingen (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 071 216 B1

**Beschreibung**

Die vorliegende Erfindung betrifft Pyrrolidinderivate. Im Speziellen betrifft sie Pyrrolidinderivate der allgemeinen Formel

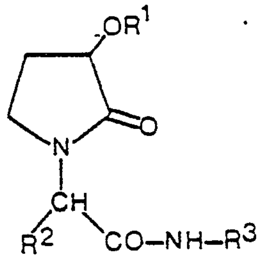

(I)

worin $R^1$ Wasserstoff oder niederes Alkanoyl, $R^2$ Wasserstoff oder niederes Alkyl und $R^3$ Wasserstoff, niederes Alkyl oder einen Rest der Formel $—(CH_2)_n—NR^4R^5$, n eine ganze Zahl von 2 bis 4 und $R^4$ und $R^5$ je Wasserstoff oder niederes Alkyl oder zusammen mit dem Stickstoffatom einen gegebenenfalls durch eine oder zwei niedere Alkylgruppen substitiuierten Pyrrolidin-, Piperidin-, Piperazin- oder Morpholinrest bedeuten, und Säureadditionssalze von Verbindungen der allgemeinen Formel I, welche basischen Charakter aufweisen.

Diese Verbindungen sind neu und zeichnen sich durch wertvolle pharmakodynamische Eigenschaften aus.

Aus GB-A-1 309 692, U.S.-A-4.145.347 und CH-A-436 304 sind 2-Oxo-1-pyrrolidinyl-carbonsäureamide bekannt, welche im Pyrrolidonring keine Hydroxy- oder Alkanoyloxygruppe enthalten.

Aus DE-A-2 759 033 sind an ihrer Hydroxy-Gruppe gegebenenfalls durch eine Trialkylsilylgruppe geschützte 4-Hydroxy-2-oxo-1-pyrrolidinyl-carbonsäureester bekannt.

Aus EP-A-24 030 sind Derivate von 1-(4'-Methoxybenzoyl)-2-pyrrolidinon bekannt, welche in 3-, 4- oder 3'-Stellung eine gegebenenfalls geschützte Hydroxygruppe aufweisen.

Gegenstand der vorliegenden Erfindung sind Verbindungen der allgemeinen Formel I und Säureadditionssalze von Verbindungen der allgemeinen Formel I, welche basischen Charakter aufweisen, als solche und als pharmazeutische Wirkstoffe, die Herstellung dieser Verbindungen, Zwischenprodukte für die Herstellung dieser Verbindungen, ferner diese enthaltende Arzneimittel und die Herstellung solcher Arzneimittel, sowie die Verwendung von Verbindungen der allgemeinen Formel I, und von Säureadditionssalzen von Verbindungen der allgemeinen Formel I, welche basischen Charakter aufweisen, bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung der intellektuellen Leistungsfähigkeit.

Der in der vorliegenden Beschreibung verwendete Ausdruck « niederes Alkyl » bezeichnet geradkettige oder verzweigte, gesättigte Kohlenwasserstoffreste, welche höchstens 7, vorzugsweise höchstens 4 Kohlenstoffatome enthalten, wie Methyl, Aethyl, n-Propyl, Isopropyl, n-Butyl, Isobutyl, s-Butyl, t-Butyl und dergleichen. Der Ausdruck « niederes Alkanoyl » bezeichnet geradkettige oder verzweigte, gesättigte Fettsäurereste, welche höchstens 8, vorzugsweise höchstens 4 Kohlenstoffatome enthalten, wie Formyl, Acetyl, Propionyl, Butyryl, Isobutyryl und dergleichen.

Die Verbindungen der allgemeinen Formel I enthalten mindestens ein asymmetrisch substituiertes Kohlenstoffatom ; die vorliegende Erfindung umfasst sowohl die optisch einheitlichen enantiomeren Formen dieser Verbindungen, als auch Mischungen davon (insbesondere die Racemate).

In einem bevorzugten Aspekt umfasst die vorliegende Erfindung Verbindungen der allgemeinen Formel I, worin $R^1$ und $R^2$ Wasserstoff und $R^3$ Wasserstoff, 2-(Diisopropyl-amino)äthyl oder 2-(2,6-Dimethyl-1-piperidinyl)äthyl bedeuten.

Ganz besonders bevorzugte Verbindungen der allgemeinen Formel I sind :

(R/S)-cis-N-[2-(2,6-Dimethyl-1-piperidinyl)äthyl]-2-(3-hydroxy-2-oxo-1-pyrrolidinyl)acetamid und (R/S)-2-(3-Hydroxy-2-oxo-1-pyrrolidinyl)acetamid.

Weitere bevorzugte Verbindungen der allgemeinen Formel I sind :

(R)-cis-N-[2-(2,6-Dimethyl-1-piperidinyl)äthyl]-2-(3-hydroxy-2-oxo-1-pyrrolidinyl)acetamid, (S)-cis-N-[2-(2,6-Dimethyl-1-piperidinyl)äthyl]-2-(3-hydroxy-2-oxo-1-pyrrolidinyl)acetamid, (R)-2-(3-Hydroxy-2-oxo-1-pyrrolidinyl)acetamid, (S)-2-(3-Hydroxy-2-oxo-1-pyrrolidinyl)acetamid und (R/S)-N-[2-(Diisopropylamino)äthyl]-2-(3-hydroxy-2-oxo-1-pyrrolidinyl)acetamid.

Die Verbindungen der allgemeinen Formel I und, sofern sie basischen Charakter aufweisen, ihre pharmazeutisch annehmbaren Säureadditionssalze können erfindungsgemäss dadurch hergestellt

werden, dass man

a) eine Verbindung der allgemeinen Formel

$$H_2R—R^3 \qquad II$$

Worin $R^3$ obige Bedeutung besitzt, mit einer Carbonsäure der allgemeinen Formel

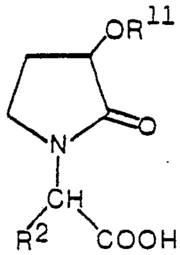

(III)

worin $R^2$ obige Bedeutung besitzt, und $R^{11}$ Wasserstoff oder niederes Alkanoyl oder — falls $R^3$ in Formel II Wasserstoff bedeutet — auch eine andere mittels Ammoniak abspaltbare Gruppe bedeutet, oder mit einem reaktionsfähigen funktionellen Derivat davon umsetzt, oder

b) eine Verbindung der allgemeinen Formel

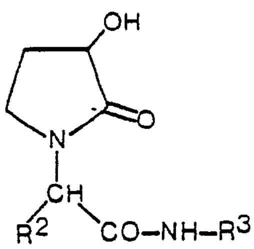

(Ib)

worin $R^2$ und $R^3$ obige Bedeutung besitzen, mit einem einen niederen Alkanoylrest liefernden Mittel acyliert, oder

c) aus einer Verbindung der allgemeinen Formel

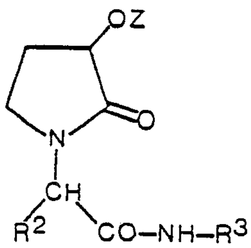

(IV)

worin $R^2$ und $R^3$ obige Bedeutung besitzen, und Z eine Schutzgruppe bedeutet, die mit Z bezeichnete Schutzgruppe abspaltet, und

d) erwünschtenfalls ein erhaltenes Gemisch von Diastereoisomeren in die entsprechenden Racemate auftrennt, und/oder

e) erwünschtenfalls ein erhaltenes Racemat einer Verbindung der allgemeinen Formel I, worin $R^1$ Wasserstoff und/oder $R^3$ einen basischen Rest bedeuten, in die optischen Antipoden spaltet, und/oder

f) erwünschtenfalls eine Verbindung der allgemeinen Formel I, welche basischen Charakter aufweist, in ein pharmazeutisch annehmbares Säureadditionssalz überführt.

Gemäss Verfahrensvariante a) können Verbindungen der Formel I hergestellt werden, indem man ein Amin der Formel II mit einer Carbonsäure der Formel III oder einem reaktionsfähigen funktionellen Derivat davon umsetzt. Verwendet man die freie Carbonsäure der Formel III, so arbeitet man zweckmässigerweise in einem inerten organischen Lösungsmittel und in Gegenwart eines Kondensationsmittels. Geeignete inerte organische Lösungsmittel sind beispielsweise Aether, wie Tetrahydrofuran, Diäthyläther, t-Butylmethyläther, Dioxan, Aethylenglykol-dimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen. Geeignete Kondensationsmittel sind beispielsweise Dicyclohexylcarbodiimid, gegebenenfalls zusammen mit N-Hydroxysuccinimid, 1-(niederes Alkyl)-2-halogen-pyridiniumsalze und

dergleichen. Man arbeitet dabei in einem Temperaturbereich von etwa 0 °C bis Siedetemperatur des Reaktionsgemisches, zweckmässigerweise jedoch bei Raumtemperatur.

Verwendet man für die obige Reaktion ein reaktionsfähiges funktionelles Derivat einer Carbonsäure der Formel III, so kommen in erster Linie entsprechende Carbonsäureester, insbesondere niedere Alkylester, wie die Methyl- und Aethylester, entsprechende Carbonsäurehalogenide, insbesondere die Carbonsäurechloride, entsprechende Carbonsäureanhydride und gemischte Anhydride (z. B. mit Mesitylensulfonsäure, Ameisensäureäthylester und dergleichen), entsprechende Carbonsäureimidazolide und dergleichen in Betracht. Die reaktionsfähigen funktionellen Derivate der Carbonsäuren der Formel III müssen nicht in allen Fällen isoliert, sondern können *in situ* hergestellt und unmittelbar weiterverarbeitet werden. Zweckmässigerweise arbeitet man in einem inerten organischen Lösungsmittel, wobei insbesondere die bereits oben erwähnten in Frage kommen. Je nach Reaktivität des verwendeten Carbonsäurederivates arbeitet man in einem Temperaturbereich von etwa 0 °C bis Siedetemperatur des Reaktionsgemisches.

Verwendet man die freie Carbonsäure der Formel III in Gegenwart eines reaktiven Kondensationsmittels oder besonders reaktive funktionelle Derivate davon, wie z. B. entsprechende Carbonsäurehalogenide oder -anhydride, so kommen als Ausgangsstoffe nur solche Verbindungen in Frage, worin $R^{11}$ nicht Wasserstoff bedeutet.

In einer bevorzugten Ausführungsform verwendet man einen niederen Alkylester einer Carbonsäure der Formel III und überschüssiges Amin der Formel II als Lösungsmittel. Wird ein am Stickstoff unsubstituiertes Amid der Formel I gewünscht, so handelt es sich bei der Verbindung der Formel II um Ammoniak, welcher vorzugsweise in wässriger oder alkoholischer (insbesondere methanolischer) Lösung eingesetzt wird. Dabei gilt es zu beachten, dass bei Verwendung eines Ueberschusses an Ammoniak eine allenfalls im Ausgangsprodukt der Formel III vorhandene niedere Alkanoylgruppe abgespalten wird, wobei man eine Verbindung der Formel I erhält, worin $R^1$ Wasserstoff bedeutet. Dasselbe gilt, wenn $R^{11}$ im Ausgangsprodukt der Formel III eine andere mittels Ammoniak abspaltbare Gruppe ist. Bei den mittels Ammoniak abspaltbaren Gruppen handelt es sich in erster Linie um Acylgruppen, z. B. um Alkanoylgruppen (wie die bereits erwähnten niederen Alkanoylgruppen), um durch Halogen, Alkoxygruppen oder Aryloxygruppen o.dgl. substituierte Alkanoylgruppen (wie Chloracetyl, Trifluoracetyl, Methoxyacetyl, Phenoxyacetyl usw.), um gegebenenfalls durch Halogen o.dgl. substituierte Alkoxycarbonyl- oder Aralkyloxycarbonylgruppen (wie Benzyloxycarbonyl, Trichloräthoxycarbonyl, Tribromäthoxycarbonyl usw.), um Aroylcarbonylgruppen (wie Benzoylformyl usw.), um Acylreste optisch aktiver Säuren, wie (3-Oxo-4,7,7-trimethyl-2-oxabicyclo-[2.2.1]hept-1-yl)carbonyl usw.

Gemäss Verfahrensvariante b) können Verbindungen der Formel I, worin $R^1$ niederes Alkanoyl bedeutet, dadurch hergestellt werden, dass man eine Verbindung der Formel Ib mit einem einen niederen Alkanoylrest liefernden Mittel behandelt. Geeignete, einen niederen Alkanoylrest liefernde Mittel sind beispielsweise niedere Alkancarbonsäurehalogenide, insbesondere die Chloride, entsprechende Anhydride und gemischte Anhydride (beispielsweise mit den bereits oben erwähnten Säuren), entsprechende niedere Alkancarbonsäureimidazolide und dergleichen. Zweckmässigerweise arbeitet man in einem inerten organischen Lösungsmittel, wobei insbesondere Aether, wie Tetrahydrofuran, Diäthyläther, t-Butylmethyläther, Dioxan, Aethylenglykoldimethyläther oder dergleichen, halogenierte Kohlenwasserstoffe, wie Methylenchlorid, Chloroform, 1,2-Dichloräthan oder dergleichen, aromatische Kohlenwasserstoffe, wie Toluol und dergleichen, Acetonitril, Dimethylformamid oder dergleichen in Frage kommen. Die Reaktionstemperatur liegt zweckmässigerweise in einem Bereich von etwa 0 °C bis Siedetemperatur des Reaktionsgemisches.

Gemäss Verfahrensvariante c) können Verbindungen der Formel I, worin $R^1$ Wasserstoff bedeutet, dadurch hergestellt werden, dass man aus einer Verbindung der Formel IV die mit Z bezeichnete Schutzgruppe abspaltet. Als Schutzgruppen in den Verbindungen der Formel IV eignen sich natürlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Als derartige Schutzgruppen eignen sich beispielsweise leicht abspaltbare metallorganische Gruppen, insbesondere Trialkylsilylgruppen, wie die Trimethylsilyl-, die t-Butyldimethylsilylgruppe und dergleichen leicht abspaltbare Acetal- und Ketalschutzgruppen, wie die Tetrahydropyran-2-yl-, die 4-Methoxy-tetrahydropyran-4-ylgruppe und dergleichen, die Benzylgruppe usw. Die Entfernung der Schutzgruppe aus den Verbindungen der Formel IV erfolgt nach an sich bekannten Methoden, wobei natürlich für die Wahl der zur Anwendung gelangenden Methode die Natur der zu entfernenden Schutzgruppe in Betracht gezogen werden muss und zu beachten ist, dass nur die Schutzgruppe selektiv entfernt, andere im Molekül vorhanden Strukturelemente jedoch nicht angegriffen werden sollen. Die Trimethylsilylgruppe kann beispielsweise durch Behandeln mit verdünnter Salzsäure in Tetrahydrofuran oder durch Erhitzen in wässrigem Aethanol oder Methanol auf Siedetemperatur abgespalten werden. Die oben erwähnten Acetal- und Ketalschutzgruppen können unter milden sauren wässrigen Bedingungen (z. B. mittels 0,1N Salzsäure) oder durch Umacetalisieren mit einem niederen Alkanol, wie Methanol oder Aethanol, in Gegenwart eines sauren Katalysators, wie Salzsäure, Pyridinium-p-toluolsulfonat, p-Toluolsulfonsäure oder dergleichen, abgespalten werden. Die Benzylgruppe kann beispielsweise hydrogenolytisch abgespalten werden, z. B. mit einem gegebenenfalls an einen Träger gebundenen Katalysator, wie Platin, Platinoxid, Palladium oder dergleichen.

Die Auftrennung eines erhaltenen Gemisches von Diastereoisomeren in die entsprechenden Racemate erfolgt nach an sich bekannten und jedem Fachmann geläufigen Methoden. Die gewünschte Auftrennung kann beispielsweise mittels chromatographischer Methoden erfolgen.

Racemate von Verbindungen der Formel I, worin $R^1$ Wasserstoff und/oder $R^3$ einen basischen Rest bedeuten, können dadurch gespalten werden, dass man diese Verbindungen mit einer optisch aktiven Carbonsäure, wie Weinsäure, (+)-Di-O,O'-p-toluoyl-D-weinsäure, (—)-(3-Oxo-4,7,7-trimethyl-2-oxabicyclo[2.2.1]hept-1-yl)carbonsäure, oder dergleichen verestert bzw. mit einer solchen Säure ein Salz bildet, und anschliessend die so erhaltenen diastereoisomeren Verbindungen bzw. Salze, beispielsweise durch fraktionierte Kristallisation oder mittels chromatographischer Methoden, auftrennt und die optisch einheitlichen Verbindungen der Formel I durch Esterspaltung bzw. durch Behandeln mit einer Base freisetzt.

Die Herstellung von pharmazeutisch annehmbaren Säureadditionssalzen von Verbindungen der Formel I erfolgt nach allgemein üblichen und jedem Fachmann geläufigen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate, Tartrate, Citrate, Maleinate, Ascorbate, Acetate und dergleichen.

Wie eingangs erwähnt sind Zwischenprodukte für die Herstellung der Verbindungen der Formel I ebenfalls Gegenstand der vorliegenden Erfindung, und zwar diejenigen der obigen Formeln III und IV, sofern die Schutzgruppe keine niedere Alkanoylgruppe ist.

Niedere Alkylester von Verbindungen der Formel III können beispielsweise dadurch hergestellt werden, dass man ein Pyrrolidinon-Derivat der allgemeinen Formel

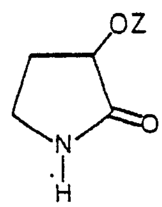

(V)

worin Z obige Bedeutung besitzt, mit einer zur Abstraktion des Wasserstoffatoms am Stickstoffatom in 1-Stellung befähigten Base (z. B. mit Natriumhydrid) behandelt und hierauf das erhaltene Anion mit einer Verbindung der allgemeinen Formel

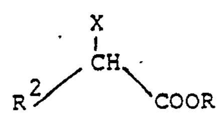

(VI)

worin $R^2$ obige Bedeutung besitzt, X ein Halogenatom und R niederes Alkyl bedeuten, umsetzt. Man erhält somit eine Verbindung der allgemeinen Formel

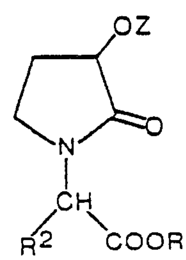

(VII)

worin $R^2$, R und Z obige Bedeutung besitzen. Verbindungen der Formel VII, worin Z niederes Alkanoyl bedeutet, sind niedere Alkylester von Verbindungen der Formel III, worin $R^{11}$ niederes Alkanoyl bedeutet. Niedere Alkylester von Verbindungen der Formel III, worin $R^{11}$ Wasserstoff bedeutet, können erhalten werden, indem man aus einer Verbindung der Formel VII die mit Z bezeichnete Schutzgruppe abspaltet.

Carbonsäuren der Formel III, worin $R^{11}$ Wasserstoff bedeutet, d. h. Verbindungen der allgemeinen Formel

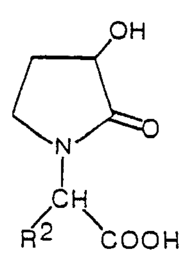

(IIIa)

worin $R^2$ obige Bedeutung besitzt, können erhalten werden, indem man in einer Verbindung der Formel VII die Estergruppe hydrolysiert, und vorgängig, anschliessend ober in gleichen Arbeitsgang die mit Z bezeichnete Schutzgruppe abspaltet. Carbonsäuren der Formel III, worin $R^{11}$ niederes Alkanoyl bedeutet, können erhalten werden, indem man eine Verbindung der Formel IIIa mit einem einen niederen Alkanoylrest liefernden Mittel umsetzt, beispielsweise durch Behandeln mit einem niederen Alkancarbonsäureanhydrid oder -chlorid. Andere Reste, wie (3-Oxo-4,7,7-trimethyl-2-oxabicyclo[2.2.1]hept-1-yl)carbonyl, können analog eingeführt werden, beispielsweise also mittels (3-Oxo-4,7,7-trimethyl-2-oxabicyclo[2.2.1]-hept-1-yl)carbonylchlorid.

Die als Ausgangsstoffe verwendeten Verbindungen der Formel IV können dadurch erhalten werden, dass man eine Verbindung der Formel VII mit einem Amin der Formel II umsetzt, und zwar in Analogie zur Verfahrensvariante a). Es gilt zu beachten, dass dabei nur solche Schutzgruppen in Betracht kommen, welche unter diesen Reaktionsbedingungen nicht angegriffen werden. Besonders geeignete Schutzgruppen sind beispielsweise die bereits früher erwähnten Silylschutzgruppen und die Benzylgruppe.

Die Verbindungen der Formel V können ihrerseits beispielsweise aus 3-Hydroxy-2-pyrrolidinon hergestellt werden, und zwar durch Einführung der gewünschten Schutzgruppe ; die Methoden zur Einführung der Schutzgruppe variieren je nach deren Natur, sind jedoch jedem Fachmann geläufig.

Gewisse Verbindungen der allgemeinen Formel V können auch aus 4-Amino-2-hydroxybuttersäure hergestellt werden, und zwar durch Methoden, welche in einem Arbeitsgang Cyclisation und Einführung der gewünschten Schutzgruppe bewirken. So kann man beispielsweise zu 3-(Trimethylsilyloxy)-2-pyrrolidinon gelangen, indem man 4-Amino-2-hydroxybuttersäure in Gegenwart geringer Mengen von Trimethylchlorsilan mit Hexamethyldisilazan oder mit bis-(Trimethylsilyl)harnstoff oder mit bis-(Trimethylsilyl)acetamid umsetzt.

Die Verbindungen der Formeln III und IV weisen in 3-Stellung des 5-gliedrigen Heterocyclus ein asymmetrisch substituiertes Kohlenstoffatom auf. Die diesbezüglichen stereochemischen Verhältnisse bestimmen die stereochemischen Verhältnisse bei den aus den Verbindungen der Formeln III und IV hergestellten Verbindungen der Formel I. Die stereochemischen Verhältnisse in 3-Stellung des 5-gliedrigen Heterocyclus der Verbindungen der Formeln III und IV werden ihrerseits durch die bei der Herstellung dieser Verbindungen verwendeten Vorprodukte und/oder Methoden bestimmt.

Wie eingangs erwähnt, sind die Pyrrolidinderivate der Formel I neue Verbindungen mit äusserst wertvollen pharmakodynamischen Eigenschaften. Sie weisen nur eine geringe Toxizität auf, und es hat sich gezeigt, dass sie in dem nachstehend beschriebenen Tierversuch experimentell hervorgerufener cerebraler Insuffizienz entgegenzuwirken vermögen.

Die Test-Apparatur ist eine « Skinner box » mit einem elektrifizierbaren Gitterboden (30 × 40 cm) und einer grauen Plastik-Plattform (15 × 15 × 0,8 cm) in der Ecke vorne rechts. Unerfahrene männliche Ratten (100-120 g) werden einzeln auf die Plattform gebracht. Sobald sie auf den Gitterboden hinabsteigen, erhalten sie einen elektrischen Fuss-Schock (0,8 mA). Die normale Reaktion unerfahrener Ratten ist, daraufhin auf die Plattform zurückzuspringen. Da jedoch die Ratten immer nochmals herabzuklettern versuchen, muss die Fuss-Schock-Prozedur für jedes Tier drei- bis fünfmal wiederholt werden. Nach diesen drei bis fünf Wiederholungen pro Tier haben die Ratten eine sogenannte « passive avoidance response » erlernt, d. h. sie versuchen nicht mehr, auf den Gitterboden hinabzusteigen, weil sie wissen, dass sie bestraft werden.

Unmittelbar anschliessend werden drei Gruppen von je 30 Tieren gebildet. Die erste Gruppe erhält eine Injektion (i. p.) von 0,3 mg/kg Scopolamin sowie destilliertes Wasser (2 ml/kg p. o.). Die zweite Gruppe erhält eine Injection (i. p.) von 0,3 mg/kg Scopolamin und eine orale Dosis der Testsubstanz. Die dritte Gruppe erhält ausschliesslich destilliertes Wasser (p. o.).

2 Stunden später wird jede Ratte einmal auf die Plattform in der « Skinner box » gesetzt. Das Kriterium für die Beurteilung dieses Tests zur Ermittlung einer Präparatwirkung auf das Kurzzeit-Gedächtnis ist, ob das Tier während 60 Sekunden auf der Plattform bleibt oder nicht (das Ergebnis kann also für jedes Tier nur « ja » oder « nein » lauten). Die statistische Signifikanz der Unterschiede zwischen den bei der ersten und der zweiten Gruppe erhaltenen Resultaten wird mittels des Chi-Quadrat-Tests ermittelt.

70-75 % der lediglich mit destilliertem Wasser (p. o.) behandelten Tiere erinnern sich 2-4 Stunden nach Erlernen der « passive avoidance response » noch daran, dass sie auf der Plattform bleiben sollten. Bei 85-92 % der mit Scopolamin (0,3 mg/kg i. p.) und destilliertem Wasser (p. o.) behandelten Tiere lässt sich während 3-4 Stunden ein retrograder Effekt auf das Kurzzeitgedächtnis feststellen, d. h. sie haben vergessen, dass sie auf der Plattform bleiben müssen. Eine Substanz, welche cerebraler Insuffizienz entgegenzuwirken vermag, kann die durch die Injektion (i. p.) von 0,3 mg/kg Scopolamin hervorgerufene Blockierung des Kurzzeitgedächtnisses aufheben. Eine Dosis eines Präparats wird dann als « aktiv » gegen Scopolamin bezeichnet, wenn die Anzahl der positiven Ergebnisse (« ja ») von derjenigen mit Scopolamin (0,3 mg/kg i. p.) und nur destilliertem Wasser (p. o.) behandelter Kontroll-Tiere signifikant verschieden ist.

In der nachstehenden Tabelle ist angegeben, bei welchen Dosen bestimmte Verbindungen der Formel I in dem vorstehend beschriebenen Versuch eine signifikante Aktivität zeigen. Die Tabelle enthält ausserdem Angaben über die akute Toxizität ($DL_{50}$ in mg/kg bei einmaliger oraler Verabreichung an Mäuse).

Tabelle

| Verbindung | signifikant wirksame Dosen in mg/kg p.o. | $DL_{50}$ in mg/kg p.o. |
|---|---|---|
| A | 0,01<br>0,03<br>0,1<br>0,3<br>1,0 | >5000 |
| B | 10<br>30 | >5000 |
| C | 10<br>30<br>50 | >5000 |
| D | 10<br>30<br>50 | >5000 |
| E | 3 | >5000 |

Verbindung A: (R/S)-cis-N-[2-(2,6-Dimethyl-1-piperidinyl)-äthyl]-2-(3-hydroxy-2-oxo-1-pyrrolidinyl)-acetamid

Verbindung B: (S)-2-(3-Hydroxy-2-oxo-1-pyrrolidinyl)-acetamid

Verbindung C: (R)-2-(3-Hydroxy-2-oxo-1-pyrrolidinyl)-acetamid

Verbindung D: (R/S)-2-(3-Hydroxy-2-oxo-1-pyrrolidinyl)-acetamid

Verbindung E: (R/S)-N-[2-(Diisopropylamino)äthyl]-2-(3-hydroxy-2-oxo-1-pyrrolidinyl)acetamid

Die Verbindungen der Formel I und pharmazeutisch annehmbare Säureadditionssalze von Verbindungen der Formel I, welche basischen Charakter aufweisen, können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Wie eingangs erwähnt, sind Arzneimittel, enthaltend eine Verbindung der Formel I oder ein pharmazeutisch annehmbares Säureadditionssalz einer Verbindung der Formel I, welche basischen Charakter aufweist, ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I oder pharmazeutisch annehmbare Säureadditionssalze von Verbindungen der Formel I, welche basischen Charakter aufweisen, und erwünschtenfalls einen oder mehrere andere therapeutisch wertvolle Stoffe in eine galenische Darreichungsform bringt.

Zur Herstellung von Tabletten, Lacktabletten, Dragées und Hartgelatinekapseln kann die Verbindung der Formel I mit pharmazeutisch inerten, anorganischen oder organischen Excipientien verarbeitet werden. Als solche Excipientien kann man z. B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z. B. vegetabile Oele, Wachse, Fette, halbfeste und flüssige Polyole etc.

Zur Herstellung von Lösungen und Sirupen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin,

vegetabile Oele etc.

Für Suppositorien eignen sich als Excipientien z. B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und pharmazeutisch annehmbare Säureadditionssalze von Verbindungen der Formel I, welche basischen Charakter aufweisen, bei der Bekämpfung bzw. Verhütung der cerebralen Insuffizienz bzw. bei der Verbesserung der intellektuellen Leistungsfähigkeit verwenden, beispielsweise bei cerebralen Insulten, in der Geriatrie, bei Alkoholismus usw. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2 500 mg angemessen sein, wobei aber die soeben angegebene obere Grenze auch überschritten werden kann, wenn sich dies als angezeigt erweisen sollte.

In den nachfolgenden Beispielen, welche die vorliegende Erfindung illustrieren, deren Umfang jedoch in keiner Weise einschränken sollen, sind sämtliche Temperaturen in Celsiusgraden angegeben.

Beispiel 1

a) Zu 51,9 g (R/S)-3-Trimethylsilyloxy-2-pyrrolidinon und 136 ml Bromessigsäureäthylester in 520 ml wasserfreiem Acetonitril werden unter Rühren zwischen 45° und 50° innerhalb von 30 Minuten portionenweise 38,3 g einer ca. 55 %igen Dispersion von Natriumhydrid in Mineralöl zugegeben. Danach wird das Gemisch innerhalb von 30 Minuten unter Rühren auf Rückflusstemperatur gebracht, 1 Stunde am Rückfluss weitergerührt und dann filtriert. Das Filtrat wird eingedampft und der verbleibende Rückstand, enthaltend den (R/S)-2-(3-Trimethylsilyloxy-2-oxo-1-pyrrolidinyl)essigsäureäthylester, wird in 500 ml Tetrahydrofuran gelöst. Zu dieser Lösung werden 71 ml 1N Salzsäure und nach 15 Minuten 7,2 g Natriumhydrogencarbonat zugegeben, worauf man 7 Minuten bei Raumtemperatur rührt und danach eindampft. Der Rückstand wird dreimal mit Acetonitril extrahiert und die vereinigten Acetonitrilextrakte werden eingedampft. Der Rückstand wird an Kieselgel (Korngrösse 0,2-0,5 mm) chromatographiert. Der mit Methylenchlorid und Essigester eluierte (R/S)-2-(3-Hydroxy-2-oxo-1-pyrrolidinyl)essigsäureäthylester wird aus einem Gemisch von Essigester/n-Hexan (1 : 2) kristallisiert und zeigt dann einen Schmelzpunkt von 80,5-81°.

b) 2,50 g (R/S)-2-(3-Hydroxy-2-oxo-1-pyrrolidinyl)essigsäureäthylester werden mit 11,5 ml einer ca. 25 %igen Ammoniumhydroxidlösung versetzt, worauf während 1 Stunde bei Raumtemperatur gerührt wird. Das Reaktionsgemisch wird mit Acetonitril versetzt und eingedampft. Den Rückstand versetzt man fünfmal mit Acetonitril und dampft jeweils wieder ein. Man erhält (R/S)-2-(3-Hydroxy-2-oxo-1-pyrrolidinyl)acetamid, welches nach Umkristallisation aus Methanol/Diäthyläther (1 : 2) bei 163-164° schmilzt.

Beispiel 2

4,5 g (R/S)-2-(3-Hydroxy-2-oxo-1-pyrrolidinyl)essigsäureäthylester werden mit 7,1 g cis-2-(2,6-Dimethyl-1-piperidinyl)äthylamin 3,5 Stunden unter Stickstoff auf 100° erwärmt. Danach gibt man Diäthyläther zu, verrührt, filtriert und chromatographiert den Filterrückstand (6,5 g) an 30 g Kieselgel (Korngrösse 0,2-0,5 mm). Das mit Methylenchlorid und Methanol eluierte (R/S)-cis-N-[2-(2,6-Dimethyl-1-piperidinyl)äthyl]-2-(3-hydroxy-2-oxo-1-pyrrolidinyl)acetamid zeigt nach Umkristallisation aus Essigester einen Schmelzpunkt von 131-132°.

Beispiel 3

4,5 g (R/S)-2-(3-Hydroxy-2-oxo-1-pyrrolidinyl)essigsäureäthylester werden mit 6,1 g 2-(Diisopropylamino)-äthylamin unter Stickstoff 3,5 Stunden auf 100° erwärmt. Das rohe Reaktionsprodukt wird durch Aluminiumoxid (neutral, Aktivitätsstufe III) chromatographiert. Mit Essigester/Aethylalkohol (Gemisch 1 : 1) und mit Aethylalkohol eluiert man (R/S)-N-[2-(Diisopropylamino)äthyl]-2-(3-hydroxy-2-oxo-1-pyrrolidinyl)acetamid. Dieses zeigt nach Verrühren in Diäthyläther einen Schmelzpunkt von 91-93° ; Siedepunkt 230-250°/0,01 mmHg (Kugelrohr).

Beispiel 4

a) Zu einer Suspension von 34,0 g (R)-4-Amino-2-hydroxybuttersäure in 340 ml wasserfreiem o-Xylol werden 89 ml Hexamethyldisilazan und 0,6 ml Trimethylchlorsilan zugegeben. Die Mischung wird während 4 Stunden unter Rühren zum Sieden erhitzt und dann eingedampft. Der Rückstand wird viermal mit Toluol extrahiert. Die vereinigten Toluolextrakte werden im Vakuum eingedampft, worauf der Rückstand in 3 Portionen destilliert wird. Man erhält (R)-3-(Trimethylsilyloxy)-2-pyrrolidinon vom Siedepunkt 90-100°/0,01 mmHg (Kugelrohr).

b) Die Verfahrensweise von Beispiel 1, Absatz a) wird mit dem Unterschied wiederholt, dass als Ausgangsprodukt das (R)-3-(Trimethylsilyloxy)-2-pyrrolidinon eingesetzt wird. Man erhält nach Umkristallisation aus Essigester/n-Hexan (R)-(+)-2-(3-Hydroxy-2-oxo-1-pyrrolidinyl)essigsäureäthylester vom Schmelzpunkt 84-85° ; $[\alpha]_D^{20}$ : +68°, $[\alpha]_{546}^{20}$ : +82°, $[\alpha]_{365}^{20}$ : +255° (Dimethylformamid, c = 1,0).

c) Aus dem (R)-(+)-2-(3-Hydroxy-2-oxo-1-pyrrolidinyl)-essigsäureäthylester erhält man nach dem in Absatz b) von Beispiel 1 beschriebenen Verfahren das (R)-(+)-2-(3-Hydroxy-2-oxo-1-pyrrolidinyl)acetamid mit einem Schmelzpunkt von 197-198° ; $[\alpha]_D^{20}$ : +81°, $[\alpha]_{546}^{20}$ : +97°, $[\alpha]_{365}^{20}$ : +308° (Dimethylformamid, c = 1,0).

Beispiel 5

Aus dem (R)-(+)-2-(3-Hydroxy-2-oxo-1-pyrrolidinyl)-essigsäureäthylester erhält man nach dem in Beispiel 2 beschriebenen Verfahren das (R)-(+)-cis-N-[2-(2,6-Dimethyl-1-piperidinyl)äthyl]-2-(3-hydroxy-2-oxo-1-pyrrolidinyl)acetamid mit einem Schmelzpunkt von 101-102° ; $[\alpha]_D^{20}$ : +43°, $[\alpha]_{546}^{20}$ : +52°, $[\alpha]_{365}^{20}$ : +162° (Acetonitril, c = 1,00).

Beispiel 6

a) Analog zum Verfahren gemäss Absatz a) von Beispiel 4 erhält man aus (S)-(—)-4-Amino-2-hydroxybuttersäure den (S)-(—)-2-(3-Hydroxy-2-oxo-1-pyrrolidinyl)essigsäureäthylester vom Schmelzpunkt 85-85,5° ; $[\alpha]_D^{20}$ : — 69°, $[\alpha]_{546}^{20}$ : — 84°, $[\alpha]_{365}^{20}$ : — 259° (Chloroform, c = 1,0).

b) Nach dem in Absatz b) von Beispiel 1 beschriebenen Verfahren erhält man aus (S)-(—)-2-(3-Hydroxy-1-pyrrolidinyl)essigsäureäthylester das (S)-(—)-2-(3-Hydroxy-2-oxo-1-pyrrolidinyl)acetamid mit einem Schmelzpunkt von 197-198° ; $[\alpha]_D^{20}$ : — 82°, $[\alpha]_{546}^{20}$ : — 99°, $[\alpha]_{365}^{20}$ : — 313° (Dimethylformamid c = 1,00).

Beispiel 7

Aus dem (S)-(—)-2-(3-Hydroxy-2-oxo-1-pyrrolidinyl)-essigsäureäthylester erhält man in Analogie zu dem in Beispiel 2 beschriebenen Verfahren das (S)-(—)-cis-N-[2-(2,6-Dimethyl-1-piperidinyl)äthyl]-2-(3-hydroxy-2-oxo-1-pyrrolidinyl)acetamid mit einem Schmelzpunkt von 101-103° ; $[\alpha]_D^{20}$ : — 44°, $[\alpha]_{545}^{20}$ : — 53°, $[\alpha]_{365}^{20}$ : — 165° (Acetonitril, c = 1,0).

Beispiel 8

1,0 g (R/S)-2-(3-Hydroxy-2-oxo-1-pyrrolidinyl)-essigsäureäthylester werden mit 10 ml einer 40 %igen Lösung von Methylamin in Wasser versetzt. Nach 1-stündigem Rühren bei Raumtemperatur wird das Reaktionsgemisch eingedampft. Der Rückstand wird zur Entfernung des Wassers fünfmal mit Acetonitril geschüttelt, worauf jeweils wieder eingedampft wird. Der Rückstand wird mit Diäthyläther versetzt. Durch Filtration isoliert man N-Methyl-2-(3-hydroxy-2-oxo-1-pyrrolidinyl)acetamid vom Schmelzpunkt 129-130,5°.

Beispiel 9

Ein Gemisch von 3,5 g (R/S)-2-(3-Hydroxy-2-oxo-1-pyrrolidinyl)essigsäureäthylester und 11,2 g 1,2-Aethylendiamin wird 20 Stunden bei Raumtemperatur stehengelassen. Das überschüssige 1,2-Aethylendiamin wird im Vakuum abgedampft. Der Rückstand wird in 40 ml heissem Acetonitril gelöst, worauf man 2 Stunden bei Raumtemperatur und dann 1 Stunde im Eisbad rührt. Durch Filtration isoliert man N-[2-(Amino)äthyl]-2-(3-hydroxy-2-oxo-1-pyrrolidinyl)-acetamid vom Schmelzpunkt 110-112°.

Beispiel 10

3,0 g (R/S)-2-(3-Hydroxy-2-oxo-1-pyrrolidinyl)essigsäureäthylester und 5,9 g cis-4-(2,6-Dimethyl-1-piperidinyl)butylamin werden 3,5 Stunden under Stickstoff auf 95-100° erwärmt. Das Reaktionsgemisch wird an 70 g Aluminiumoxid (Aktivitätsstufe III, neutral) chromatographiert. Das mit Aethylalkohol eluierte (R/S)-cis-N-[4-(2,6-Dimethyl-1-piperidinyl)butyl]-2-(3-hydroxy-2-oxo-1-pyrrolidinyl)acetamid zeigt nach Kristallisation aus Diäthyläther einen Schmelzpunkt von 90-92°.

Beispiel 11

3,0 g (R/S)-2-(3-Hydroxy-2-oxo-1-pyrrolidinyl)essigsäureäthylester und 2,87 g N-(2-Aminoäthyl)piperidin werden 4 Stunden unter Stickstoff auf 100° erwärmt. Das überschüssige N-(2-Aminoäthyl)piperidin wird im Hochvakuum abdestilliert. Das als Rückstand verbleibende (R/S)-N-[2-(1-Piperidinyl)äthyl]-2-(3-hydroxy-2-oxo-1-pyrrolidinyl)-acetamid zeigt nach Kristallisation aus Essigester einen Schmelzpunkt von 110-111°.

Beispiel 12

4,0 g (R/S)-2-(3-Hydroxy-2-oxo-1-pyrrolidinyl)essigsäureäthylester und 6,1 g N-(3-Aminopropyl)morpholin werden 3,5 Stunden unter Stickstoff auf 95-100° erwärmt. Das Reaktionsgemisch wird an 50 g Kieselgel (Korngrösse 0,2-0,5 mm) chromatographiert. Das mit Aethylalkohol eluierte (R/S)-N-[3-(1-Morpholinyl)propyl]-2-(3-hydroxy-2-oxo-1-pyrrolidinyl)acetamid zeigt nach Umkristallisation aus Essigester einen Schmelzpunkt von 94-96°.

Beispiel 13

3,0 g (R/S)-2-(3-Hydroxy-2-oxo-1-pyrrolidinyl)essigsäureäthylester und 3,7 g 2-(Diäthylamino)äthylamin werden 3,5 Stunden unter Stickstoff auf 95-100° erwärmt. Das Reaktionsgemisch wird an 45 g Aluminiumoxid (neutral, Aktivitätsstuffe III) chromatographiert. Das mit Methylenchlorid und Essigester eluierte rohe (R/S)-N-[2-(Diäthylamino)äthyl]-2-(3-hydroxy-2-oxo-1-pyrrolidinyl)acetamid wird nochmals durch 25 g Aluminiumoxid (neutral, Aktivitätsstufe III) chromatographiert. Das mit Essigester und Aethylalkohol eluierte Produkt wird im Kugelrohr bei ca. 250°/0,03 mmHg destilliert.

Beispiel 14

a) 15,0 g (R/S)-2-(3-Hydroxy-2-oxo-1-pyrrolidinyl)essigsäureäthylester werden in 150 ml abs. Alkohol gelöst und mit 1,95 g Natrium, gelöst in 38 ml abs. Alkohol, versetzt. 1,5 ml ionenfreies Wasser werden zugegeben, worauf über Nacht bei Raumtemperatur gerührt wird. Zur entstandenen Suspension werden 250 ml Diäthyläther zugegeben, und danach wird filtriert. Man erhält das (R/S)-2-(3-Hydroxy-2-oxo-1-pyrrolidinyl)essigsäure-natriumsalz. Das Salz wird in 200 ml Acetonitril und 12 ml 25 %iger Salzsäure über Nacht bei Raumtemperatur gerührt. Hierauf wird eingedampft. Den Eindampfrückstand versetzt man zweimal mit Acetonitril und dampft jeweils wieder ein. Der Rückstand wird in 320 ml Acetonitril am Rückfluss gekocht, worauf man heiss filtriert und das Filtrat im Eisbad 3 Stunden rührt. Durch Filtration isoliert man (R/S)-2-(3-Hydroxy-2-oxo-1-pyrrolidinyl)essigsäure vom Schmelzpunkt 153-154°.

b) Zu 1,0 g (R/S)-2-(3-Hydroxy-2-oxo-1-pyrrolidinyl)-essigsäure und 1,03 g 94,7 %igem cis-2-[2-(2,6-Dimethyl-1-piperidinyl]äthylamin, gelöst in 20 ml Dimethylformamid, werden bei 0° 1,30 g Dicyclohexylcarbodiimid, gelöst in 6 ml Dimethylformamid, zugetropft. Man lässt 4 Tage bei Raumtemperatur rühren, versetzt das Reaktionsgemisch mit 0,23 g ionenfreiem Wasser und dampft im Wasserstrahlvakuum ein. Den Rückstand versetzt man fünfmal mit Toluol und dampft jeweils wieder ein. Die in Chloroform löslichen Anteile des Rückstands werden an 60 g Aluminiumoxid (Aktivitätsstufe III, neutral) chromatographiert. Das Alkohol-Eluat wird an 15 g Aluminiumoxid (Aktivitätsstufe III, neutral) nochmals chromatographiert. Die mit Acetonitril und Aethylalkohol eluierten Fraktionen enthalten gemäss Gaschromatogramm und Massenspektrum (R/S)-cis-N-[2-(2,6-Dimethyl-1-piperidinyl)äthyl]-2-(3-hydroxy-2-oxo-1-pyrrolidinyl)acetamid.

Beispiel 15

a) 4,0 g (R/S)-2-(3-Hydroxy-2-oxo-1-pyrrolidinyl)essigsäure, 80 ml abs. Tetrahydrofuran und 5 ml Acetylchlorid werden 4 Stunden unter Rühren am Rückfluss gekocht. Danach wird das Gemisch eingedampft. Der Rückstand wird durch Kieselgel (Korngrösse 0,2-0,5 mm) filtriert. Der Eindampfrückstand der mit Essigester eluierten Fraktionen wird in Diäthyläther aufgerührt. Durch Filtration isoliert man (R/S)-2-(3-Acetoxy-2-oxo-1-pyrrolidinyl)essigsäure vom Schmelzpunkt 95-96°.

b) 1,81 g 2-Chlor-1-methyl-pyridiniumjodid werden in 10 ml Methylenchlorid suspendiert, und dazu werden bei Raumtemperatur 1,20 g (R/S)-2-(3-Acetoxy-2-oxo-1-pyrrolidinyl)essigsäure zugesetzt. Innerhalb von 15 Minuten wird bei 0° eine Lösung von 0,98 g 94,7 %igem cis-2-(2,6-Dimethyl-1-piperidinyl)äthylamin und 3,02 ml Triäthylamin in 20 ml Methylenchlorid zugetropft, worauf während 18 Stunden bei Raumtemperatur gerührt wird. Hierauf wird eingedampft und der Rückstand an Aluminiumoxid (Aktivitätsstufe III, neutral) chromatographiert. Das mit Chloroform eluierte Material wird an Kieselgel (Korngrösse 0,2-0,5 mm) chromatographiert. Das mit Alkohol eluierte (R/S)-cis-N-[2-(2,6-Dimethyl-1-piperidinyl)äthyl]-2-(3-acetoxy-2-oxo-1-pyrrolidinyl)acetamid zeigt nach Kristallisation aus Diäthyläther einen Schmelzpunkt von 120-122°.

Beispiel 16

1,0 g (R/S)-2-(3-Acetoxy-2-oxo-1-pyrrolidinyl)essigsäure werden in 15 ml abs. Tetrahydrofuran vorgelegt, worauf man in einer Portion 0,89 g N,N'-Carbonyl-diimidazol zugibt. Man rührt bei Raumtemperatur, bis die Gas-Entwicklung beendet ist. Danach werden 0,86 g 94,7 %iges cis-2-(2,6-Dimethyl-1-piperidinyl)äthylamin zugesetzt, worauf das Gemisch über Nacht bei Raumtemperatur stehengelassen und dann eingedampft wird. Der Rückstand wird an 60 g Aluminiumoxid (Aktivitätsstufe III, neutral) chromatographiert. Das mit Methylenchlorid eluierte (R/S)-cis-N-[2-(2,6-Dimethyl-1-piperidinyl)äthyl]-2-(3-aceto-

xy-2-oxo-1-pyrrolidinyl)acetamid zeigt nach Verrühren mit Diäthyläther einen Schmelzpunkt von 121-122°.

Beispiel 17

a) 1,70 g (R/S)-2-(3-Hydroxy-2-oxo-1-pyrrolidinyl)essigsäureäthylester werden in 30 ml Methylenchlorid und 0,53 ml Acetylchlorid unter Rühren 2,5 Stunden am Rückfluss gekocht. Dann wird das Gemisch eingedampft. Der Rückstand wird im Kugelrohr destilliert. Man erhält (R/S)-2-(3-Acetoxy-2-oxo-1-pyrrolidinyl)essigsäureäthylester vom Siedepunkt 225°/0,01 mmHg.

b) 0,40 g (R/S)-2-(3-Acetoxy-2-oxo-1-pyrrolidinyl)essigsäureäthylester werden mit 45 ml einer gesättigten Lösung von Ammoniak in Methylalkohol versetzt. Man lässt 1 Stunde bei Raumtemperatur stehen und dampft dann das Reaktionsgemisch ein. Den Rückstand versetzt man viermal mit Acetonitril und dampft jeweils wieder ein. Man erhält (R/S)-2-(3-Hydroxy-2-oxo-1-pyrrolidinyl)acetamid, welches nach Umkristallisieren aus Methanol/Diäthyläther (1 : 3) bei 163-164° schmilzt.

Beispiel 18

1,0 g (R/S)-2-(3-Acetoxy-2-oxo-1-pyrrolidinyl)essigsäure wird in 20 ml Toluol und 0,54 ml Thionylchlorid 2 Stunden am Rückfluss gekocht. Nach Eindampfen des Reaktionsgemisches schüttelt man den Rückstand zweimal mit Toluol und dampft das Toluol jeweils im Vakuum ab. 0,5 g des Rückstandes, enthaltend rohes (R/S)-2-(3-Acetoxy-2-oxo-1-pyrrolidinyl)essigsäurechlorid, lässt man in einer gesättigten Lösung von Ammoniak in Methanol über Nacht stehen. Nach Abdampfen der Lösungsmittel wird der Rückstand an 5 g Kieselgel (Korngrösse 0,2-0,5 mm) chromatographiert. Im Eluat (Methylenchlorid/Methanol, Gemisch 1 : 1) kann (R/S)-2-(3-Hydroxy-2-oxo-1-pyrrolidinyl)acetamid nachgewiesen werden.

Beispiel 19

Zu 1,5 g (R/S)-2-(3-Acetoxy-2-oxo-1-pyrrolidinyl)-essigsäure, 40 ml Chloroform und 0,86 g N-Hydroxysuccinimid werden 1,64 g Dicyclohexylcarbodiimid, gelöst in 20 ml Chloroform, bei Raumtemperatur zugesetzt. Nach 4 Stunden wird der Festkörper abfiltriert, worauf das Filtrat eingeengt und nochmals filtriert wird. Das Filtrat wird eingedampft. Der Rückstand wird bei Raumtemperatur mit 40 ml einer gesättigten Lösung von Ammoniak in Methanol versetzt. Man rührt 5 Minuten bei Raumtemperatur, gibt dann 10 ml ionenfreies Wasser zu und rührt während 30 Minuten bei Raumtemperatur weiter. Der ausgefallene Festkörper wird abfiltriert. Das Filtrat wird eingedampft, der Rückstand wird in 30 ml Acetonitril gerührt, und das auskristallisierte Produkt wird noch zweimal aus Acetonitril umkristallisiert. Man erhält (R/S)-2-(3-Hydroxy-2-oxo-1-pyrrolidinyl)acetamid vom Schmelzpunkt 162-164°.

Beispiel 20

1,50 g (R/S)-2-(3-Hydroxy-2-oxo-1-pyrrolidinyl)essigsäure werden in 50 ml Chloroform und 10 ml Dimethylformamid gelöst. Dazu werden 1,08 g N-Hydroxysuccinimid zugegeben. Danach werden 2,08 g Dicyclohexylcarbodiimid, gelöst in 25 ml Chloroform, zugesetzt. Man rührt das Gemisch 4 Stunden bei Raumtemperatur und filtriert dann den ausgefallenen Festkörper ab. Das Filtrat wird auf ein Volumen von ca. 20 ml eingeengt und dann nochmals filtriert. Das Filtrat wird eingedampft, und der Rückstand mit 1,70 g 94,7 %igem cis-2-(2,6-Dimethyl-1-piperidinyl)äthylamin versetzt. Man lässt das Gemisch über Nacht bei Raumtemperatur stehen und entfernt das überschüssige cis-2-(2,6-Dimethyl-1-piperidinyl)äthylamin im Vakuum. Der Rückstand wird an 60 g Aluminiumoxid (Aktivitätsstufe I, basisch) chromatographiert. Die mit Chloroform und Alkohol eluierten Fraktionen werden eingedampft. Der Rückstand wird in 35 ml Diäthyläther/Essigester (Gemisch 2 : 1) bei Raumtemperatur verrührt. Man erhält (R/S)-cis-N-[2-(2,6-Dimethyl-1-piperidinyl)äthyl]-2-(3-hydroxy-2-oxo-1-pyrrolidinyl)acetamid, (Schmelzpunkt 116-120°), welches nach nochmaliger Chromatographie an Aluminiumoxid (Aktivitätsstufe III, neutral) und Umkristallisation aus Essigester bei 126-128° schmilzt.

Beispiel 21

1,2 ml Chlorameisensäureäthylester werden in 12 ml Chloroform vorgelegt, worauf bei —30° innerhalb von 30 Minuten 2,0 g (R/S)-2-(3-Hydroxy-2-oxo-1-pyrrolidinyl)-essigsäure und 1,74 ml Triäthylamin, gelöst in 50 ml Chloroform, zugetropft werden. Nach 120-minütigem Rühren zwischen —20° und —10° werden 2,07 g 94,7 %iges cis-2-(2,6-Dimethyl-1-piperidinyl)äthylamin, gelöst in 10 ml Chloroform, zugetropft. Man lässt bei Raumtemperatur über Nacht stehen. Hierauf wird eingedampft, und der Rückstand (6 g) wird an 120 g Aluminiumoxid (Aktivitätsstufe III, neutral) chromatographiert. Das mit Acetonitril und Aethylalkohol eluierte (R/S)-cis-N-[2-(2,6-Dimethyl-1-piperidinyl)äthyl]-2-(3-hydroxy-2-oxo-1-pyrrolidinyl)acetamid zeigt nach nochmaliger Chromatographie an Aluminiumoxid und Umkristallisation aus Essigester einen Schmelzpunkt von 128-130°.

Beispiel 22

Analog dem Verfahren von Beispiel 21 erhält man aus 2 g (R/S)-2-(3-Acetoxy-2-oxo-1-pyrrolidinyl)essigsäure unter Verwendung von methanolischem Ammoniak an Stelle von cis-2-(2,6-Dimethyl-1-piperidinyl)äthylamin das (R/S)-2-(3-Hydroxy-2-oxo-1-pyrrolidinyl)acetamid. Die Reinigung erfolgt nicht durch Chromatographie, sondern durch Verrühren in Chloroform. Man erhält ein Produkt vom Schmelzpunkt 161-163°.

Beispiel 23

Analog dem Verfahren von Beispiel 21 erhält man aus 0,8 g (R/S)-2-(3-Acetoxy-2-oxo-1-pyrrolidinyl)essigsäure ein rohes Reaktionsgemisch. Dieses Gemisch wird an 40 g Aluminiumoxid (Aktivitätsstufe III, neutral) chromatographiert. Das mit Chloroform eluierte (R/S)-cis-N-[2-(2,6-Dimethyl-1-piperidinyl)äthyl]-2-(3-acetoxy-2-oxo-1-pyrrolidinyl)acetamid zeigt nach Verrühren in Diäthyläther einen Schmelzpunkt von 121-122°.

Beispiel 24

Analog dem Verfahren von Beispiel 21 erhält man aus N-(2-Aminoäthyl)pyrrolidin und (R/S)-2-(3-Hydroxy-2-oxo-1-pyrrolidinyl)essigsäure das (R/S)-N-[2-(1-Pyrrolidinyl)-äthyl]-2-(3-hydroxy-2-oxo-1-pyrrolidinyl)acetamid. Die Mikroanalyse zeigt folgende Werte :
Bruttoformel $C_{12}H_{21}O_3N_3$, MG 255,32

| | | | |
|---|---|---|---|
| Ber. : | C 56,45 % | H 8,29 % | N 16,46 % |
| Gef. : | C 56,11 % | H 8,29 % | N 16,32 % |

Beispiel 25

a) Aus (R/S)-3-Trimethylsilyloxy-2-pyrrolidinon und 2-Brompropionsäureäthylester erhält man nach dem in Absatz a) von Beispiel 1 beschriebenen Verfahren (R/S)-2-(3-Hydroxy-2-oxo-1-pyrrolidinyl)propionsäureäthylester vom Siedepunkt 200°/0,05 mmHg (Kugelrohr).

b) Nach dem in Absatz b) von Beispiel 1 beschriebenen Verfahren erhält man aus (R/S)-2-(3-Hydroxy-2-oxo-1-pyrrolidinyl)propionsäureäthylester nach chromatographischer Filtration an Kieselgel (Korngrösse 0,2-0,5 mm), Elution mit Acetonitril/Aethylalkohol (Gemisch 1 : 1) und Kristallisation aus Acetonitril das (R/S)-2-(3-Hydroxy-2-oxo-1-pyrrolidinyl)propionsäureamid vom Schmelzpunkt 139-141°.

Beispiel 26

1,5 g (R/S)-2-(3-Hydroxy-2-oxo-1-pyrrolidinyl)propionsäureäthylester werden mit 2,45 g cis-2-(2,6-Dimethyl-1-piperidinyl)äthylamin 24 Stunden bei Raumtemperatur stehengelassen. Danach wird das überschüssige cis-2-(2,6-Dimethyl-1-piperidinyl)äthylamin im Hochvakuum abdestilliert und der Rückstand (3,5 g) an 30 g Kieselgel (Korngrösse 0,2-0,5 mm) chromatographiert. Das mit Methylalkohol eluierte (R/S)-cis-N-[2-(2,6-Dimethyl-1-piperidinyl)äthyl]-2-(3-hydroxy-2-oxo-1-pyrrolidinyl)propionsäureamid zeigt nach Umkristallisation aus Essigester einen Schmelzpunkt von 129-130°.

Beispiel 27

a) Aus (R/S)-3-Trimethylsilyloxy-2-pyrrolidinon und 2-Brombuttersäureäthylester erhält man nach dem in Absatz a) von Beispiel 1 beschriebenen Verfahren (R/S)-2-(3-Hydroxy-2-oxo-1-pyrrolidinyl)buttersäureäthylester vom Siedepunkt 205°/0,02 mmHg (Kugelrohr).

b) Aus 2,05 g (R/S)-2-(3-Hydroxy-2-oxo-1-pyrrolidinyl)-buttersäureäthylester erhält man nach dem in Beispiel 2 beschriebenen Verfahren nach chromatographischer Reinigung nicht kristallisiertes (R/S)-cis-N-[2-(2,6-Dimethyl-1-piperidinyl)äthyl]-2-(3-hydroxy-2-oxo-1-pyrrolidinyl)-buttersäureamid.

Beispiel 28

Analog dem Verfahren gemäss Absatz b) von Beispiel 1 erhält man aus (R/S)-2-(3-Hydroxy-2-oxo-1-pyrrolidinyl)-buttersäureäthylester mit 25 %iger Ammoniumhydroxidlösung und Umkristallisation aus Acetonitril (R/S)-2-(3-Hydroxy-2-oxo-1-pyrrolidinyl)buttersäureamid vom Schmelzpunkt 121-122°.

Beispiel 29

a) 2,0 g (R/S)-2-(3-Hydroxy-2-oxo-1-pyrrolidinyl)essigsäureäthylester werden in 40 ml Pyridin gelöst, worauf bei 0° bis +5° portionenweise 3,54 g (—)-(3-Oxo-4,7,7-trimethyl-2-oxabicyclo[2.2.1]hept-1-

yl)carbonylchlorid ($[\alpha]_{546}^{20}$ : —23°, $CCl_4$, c = 2,0) zugegeben werden. Nach Rühren über Nacht bei Raumtemperatur wird eingedampft. Den Rückstand versetzt man viermal mit Toluol und dampft jeweils wieder ein. Der Rückstand wird an 80 g Aluminiumoxid (Aktivitätsstufe III, neutral) chromatographiert. Die mit Chloroform eluierten Fraktionen werden eingedampft, und der Rückstand wird in Diäthyläther kristallisiert. Man erhält das Diastereomerengemisch von 2-/3-[(3-Oxo-4,4,7-trimethyl-2-oxabicyclo[2.2.1]hept-1-yl)carbonyloxy]-2-oxo-1-pyrrolidinyl/essigsäureäthylester vom Schmelzpunkt 89-91°.

b) Durch Hochdruckflüssigkeitschromatographie des Diastereoisomerengemisches des 2-/3-[(3-Oxo-4,7,7-trimethyl-2-oxabicyclo[2.2.1]hept-1-yl)carbonyloxy]-2-oxo-1-pyrrolidinyl/essigsäureäthylesters an im Handel (Merck) erhältlichen, vorbepackten Hibar-Lichrosorb RT DIOL Säulen (250 × 4 mm, Korngrösse 10 μm) erreicht man unter Eluieren mit 12 % Tetrahydrofuran und 0,2 % Isopropylamin in n-Hexan die Trennung der beiden Komponenten.

Der Schmelzpunkt des (R)-2-/3-[(3-Oxo-4,4,7-trimethyl-2-oxabicyclo[2.2.1]hept-1-yl)carbonyloxy]-2-oxo-1-pyrrolidinyl/essigsäureäthylesters beträgt 107-108°, nach Kristallisation aus Benzol/n-Hexan (1 : 2).

c) 0,60 g (R)-2-/3-[(3-Oxo-4,7,7-trimethyl-2-oxabicyclo-[2.2.1]hept-1-yl)carbonyloxy]-2-oxo-1-pyrrolidinyl/essigsäureäthylester werden in 25 ml einer gesättigten Lösung von Ammoniak in Methanol 2 Stunden bei Raumtemperatur gerührt. Das Reaktionsgemisch wird eingedampft. Den Rückstand versetzt man viermal mit Acetonitril und dampft jeweils wieder ein. Durch zweimaliges Verrühren des Rückstandes in Essigester erhält man (R)-(+)-2-(3-Hydroxy-2-oxo-1-pyrrolidinyl)acetamid vom Schmelzpunkt 195-196°. $[\alpha]_D^{20}$ : +78°, $[\alpha]_{546}^{20}$ : +94°, $[\alpha]_{365}^{20}$ : +302° (Dimethylformamid, c = 1,0).

## Beispiel 30

1,5 g (R/S)-2-(3-Hydroxy-2-oxo-1-pyrrolidinyl)acetamid werden mit 13,5 ml Acetylchlorid und 25 ml Chloroform 20 Minuten zum Rückfluss erhitzt. Das Reaktionsgemisch wird eingedampft. Den Rückstand versetzt man viermal mit Toluol und dampft jeweils wieder ein. Man erhält (R/S)-2-(3-Acetoxy-2-oxo-1-pyrrolidinyl)acetamid, welches nach Kristallisation aus Diäthyläther bei 116-117° schmilzt.

## Beispiel 31

2,0 g (R/S)-cis-N-[2-(2,6-Dimethyl-1-piperidinyl)-äthyl]-2-(3-hydroxy-2-oxo-1-pyrrolidinyl)acetamid werden in 60 ml Chloroform und 1,5 ml Acetylchlorid 3 Stunden zum Rückfluss erhitzt. Nach Abdampfen der flüchtigen Anteile versetzt man den Rückstand dreimal mit Toluol und dampft jeweils im Vakuum wieder ein. Der Rückstand wird über Aluminiumoxid (Aktivitätsstufe III, neutral) chromatographiert. Das mit Chloroform extrahierte (R/S)-cis-N-[2-(2,6-Dimethyl-1-piperidinyl)äthyl]-2-(3-acetoxy-2-oxo-1-pyrrolidinyl)acetamid zeigt nach Umkristallisation aus Diäthyläther einen Schmelzpunkt von 121-122°.

## Beispiel 32

0,594 g (R/S)-cis-N-[2-(2,6-Dimethyl-1-piperidinyl)-äthyl]-2-(3-hydroxy-2-oxo-1-pyrrolidinyl)acetamid werden mit 0,222 g N-Acetylimidazol in 10 ml Toluol 4 Stunden am Rückfluss gekocht. Das Gemisch wird über 21 g Aluminiumoxid (Aktivitätsstufe III, neutral) chromatographiert. Das mit Methylenchlorid eluierte (R/S)-cis-N-[2-(2,6-Dimethyl-1-piperidinyl)äthyl]-2-(3-acetoxy-2-oxo-1-pyrrolidinyl)acetamid zeigt nach Kristallisation aus Diäthyläther einen Schmelzpunkt von 120-121°.

## Beispiel 33

Analog zum Verfahren gemäss Absatz a) von Beispiel 1 erhält man (R/S)-2-(3-Trimethylsilyloxy-2-oxo-1-pyrrolidinyl)essigsäureäthylester. Nach Behandlung mit 25 %iger Ammoniumhydroxidlösung und saurer Hydrolyse des (R/S)-2-(3-Trimethylsilyloxy-2-oxo-1-pyrrolidinyl)acetamids erhält man rohes (R/S)-2-(3-Hydroxy-2-oxo-1-pyrrolidinyl)-acetamid. Nach Chromatographie an Kieselgel (Korngrösse 0,2-0,5 mm) und Elution mit Acetonitril/Methanol (1 : 1) kann (R/S)-2-(3-Hydroxy-2-oxo-1-pyrrolidinyl)acetamid in ca. 80-90 %iger Reinheit nachgewiesen werden.

## Beispiel 34

0,3 g (R/S)-cis-N-[2-(2,6-Dimethyl-1-piperidinyl)-äthyl]-2-(3-acetoxy-2-oxo-1-pyrrolidinyl)acetamid werden in 30 ml einer gesättigten Lösung von Ammoniak in Methylalkohol 2 Stunden bei Raumtemperatur verrührt. Nach Abdampfen des Methanols versetzt man den Rückstand dreimal mit Toluol und dampft jeweils wieder ein. Nach Verrühren in Diäthyläther erhält man (R/S)-cis-N-[2-(2,6-Dimethyl-1-piperidinyl)äthyl]-2-(3-hydroxy-2-oxo-1-pyrrolidinyl)-acetamid vom Schmelzpunkt 129-130°.

## Beispiel 35

a) Zu 1,0 g (R/S)-cis-N-[2-(2,6-Dimethyl-1-piperidinyl)-äthyl]-2-(3-hydroxy-2-oxo-1-pyrrolidinyl)acetamid, gelöst in 10 ml Pyridin, werden bei 0° bis +5° portionenweise 0,86 g (—)-(3-Oxo-4,7,7-trimethyl-2-

oxabicyclo[2.2.1.]hept-1-yl)carbonylchlorid ($[\alpha]_{546}^{20}$ : —23°, $CCl_4$, c = 2,0) zugegeben. Nach 4-stündigem Rühren bei Raumtemperatur wird das Pyridin abgedampft. Den Rückstand versetzt man dreimal mit Toluol und dampft jeweils wieder ein. Der Rückstand wird an 30 g Aluminiumoxid (Aktivitätsstufe III, neutral) chromatographiert. Das Chloroformeluat enthält das Diastereoisomerengemisch von cis-N-[2-(2,6-Dimethyl-1-piperidinyl)äthyl]-2-/-3-[3-oxo-4,7,7-trimethyl-2-oxabicyclo[2.2.1]hept-1-yl)-carbonyloxy]-2-oxo-1-pyrrolidinyl/acetamid. Dieses Produkt wird ohne weitere Reinigung weiterverarbeitet.

b) Durch Hochdruckflüssigkeitschromatographie des Diastereoisomerengemisches von cis-N-[2-(2,6-Dimethyl-1-piperidinyl)äthyl]-2-/3-[(3-oxo-4,7,7-trimethyl-2-oxabicyclo[2.2.1]hept-1-yl)carbonyloxy]-2-oxo-1-pyrrolidinyl/-acetamid an im Handel (Merck) erhältlichen vorbepackten Hibar Lichrosorb RT DIOL Säulen (250 × 4 mm, Korngrösse 10 μm) bewerkstelligt man unter Eluierung mit 24 % Tetrahydrofuran und 0,4 % Isopropylamin in n-Hexan die Trennung der beiden Komponenten.

c) Aus dem (R)-cis-N-[2-(2,6-Dimethyl-1-piperidinyl)-äthyl]-2-/3-[(3-oxo-4,7,7-trimethyl-2-oxabicyclo[2.2.1]-hept-1-yl)carbonyloxy]-2-oxo-1-pyrrolidinyl/acetamid kann man durch Behandlung mit wässrigem Ammoniak (R)-cis-N-[2-(2,6-Dimethyl-1-piperidinyl)äthyl]-2-(3-hydroxy-2-oxo-1-pyrrolidinyl)acetamid erhalten. Smp. 101-102° (aus Essigester) ; $[\alpha]_D^{20}$ : +43°, $[\alpha]_{546}^{20}$ : +52°, $[\alpha]_{365}^{20}$ : +162° (Acetonitril, c = 1,00).

d) Aus dem (S)-cis-N-[2-(2,6-Dimethyl-1-piperidinyl)-äthyl]-2-/3-[(3-oxo-4,7,7-trimethyl-2-oxabicyclo[2.2.1]-hept-1-yl)carbonyloxy]-2-oxo-1-pyrrolidinyl/acetamid kann man durch Behandlung mit wässrigem Ammoniak (S)-cis-N-[2-(2,6-Dimethyl-1-piperidinyl)äthyl]-2-(3-hydroxy-2-oxo-1-pyrrolidinyl)acetamid erhalten. Smp. 101-103° (aus Essigester) ; $[\alpha]_D^{20}$ : —44°, $[\alpha]_{545}^{20}$ : —53°, $[\alpha]_{365}^{20}$ : —165° (Acetonitril, c = 1,0).

### Beispiel 36

2,97 g (R/S)-cis-N-[2-(2,6-Dimethyl-1-piperidinyl)-äthyl]-2-(3-hydroxy-2-oxo-1-pyrrolidinyl)acetamid werden in 10 ml Aethylalkohol gelöst. Dazu werden 1,26 ml einer 7,93 N Lösung von Chlorwasserstoff in Aethylalkohol zugegeben. Hierauf wird eingedampft und der Rückstand im Hochvakuum getrocknet. Man erhält (R/S)-cis-N-[2-(2,6-Dimethyl-1-piperidinyl)äthyl]-2-(3-hydroxy-2-oxo-1-pyrrolidinyl)acetamid-hydrochlorid mit dem Zersetzungspunkt 97°. Die Mikroanalyse zeigt folgende Werte :

Bruttoformel $C_{15}H_{27}N_3O_3.HCl$, MG 333,86

| | | | |
|---|---|---|---|
| Ber. : | C 53,96 | H 8.45 % | N 12,59 % |
| Gef. : | C 53,98 | H 8,57 % | N 12,40 % |

### Beispiel A

(R/S)-cis-N-[2-(2,6-Dimethyl-1-piperidinyl)äthyl]-2-(3-hydroxy-2-oxo-1-pyrrolidinyl)acetamid kann als Wirkstoff zur Herstellung von Tabletten folgender Zusammensetzung verwendet werden :

| | |
|---|---|
| Wirkstoff | 1 mg |
| Lactose | 100 mg |
| Maisstärke | 68,5 mg |
| Magnesiumstearat | 0,5 mg |
| | 170 mg |

Der feingemahlene Wirkstoff, die pulverisierte Lactose und ein Teil der Maisstärke werden gemischt. Die Mischung wird gesiebt, worauf man sie mit Maisstärkekleister verarbeitet. Anschliessend wird granuliert, getrocknet und gesiebt. Das gesiebte Granulat wird mit Magnesiumstearat gemischt, und das Gemisch wird zu Tabletten à 170 mg und geeigneter Grösse verpresst.

**Patentansprüche** (für die Vertragsstaaten : BE, CH, DE, FR, IT, LI, LU, NL, SE)

1. Pyrrolidinderivate der allgemeinen Formel

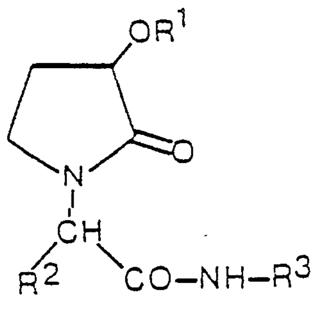

(I)

worin $R^1$ Wasserstoff oder ($C_1$-$C_8$)-Alkanoyl, $R^2$ Wasserstoff oder ($C_1$-$C_7$)-Alkyl und $R^3$ Wasserstoff, ($C_1$-$C_7$)-Alkyl oder einen Rest der Formel $-(CH_2)_n-NR^4R^5$, n eine ganze Zahl von 2 bis 4 und $R^4$ und $R^5$ je Wasserstoff oder ($C_1$-$C_7$)-Alkyl oder zusammen mit dem Stickstoffatom einen gegebenenfalls durch eine oder zwei ($C_1$-$C_7$)-Alkylgruppen substituierten Pyrrolidin-, Piperidin-, Piperazin- oder Morpholinrest bedeuten, und Säureadditionssalze von Verbindungen der Formel I, welche basischen Charakter aufweisen.

2. Verbindungen gemäss Anspruch 1, worin $R^1$ Wasserstoff, $R^2$ Wasserstoff und $R^3$ Wasserstoff, 2-(Diisopropylamino)äthyl oder 2-(2,6-Dimethyl-1-piperidinyl)äthyl bedeuten.

3. (R/S)-cis-N-[2-(2,6-Dimethyl-1-piperidinyl)äthyl]-2-(3-hydroxy-2-oxo-1-pyrrolidinyl)acetamid.

4. (R/S)-2-(3-Hydroxy-2-oxo-1-pyrrolidinyl)acetamid.

5. (R)-cis-N-[2-(2,6-Dimethyl-1-piperidinyl)äthyl]-2-(3-hydroxy-2-oxo-1-pyrrolidinyl)acetamid.

6. (S)-cis-N-[2-(2,6-Dimethyl-1-piperidinyl)äthyl]-2-(3-hydroxy-2-oxo-1-pyrrolidinyl)acetamid.

7. (R)-2-(3-Hydroxy-2-oxo-1-pyrrolidinyl)acetamid.

8. (S)-2-(3-Hydroxy-2-oxo-1-pyrrolidinyl)acetamid.

9. (R/S)-N-[2-(Diisopropylamino)äthyl]-2-(3-hydroxy-2-oxo-1-pyrrolidinyl)acetamid.

10. Carbonsäuren der allgemeinen Formel

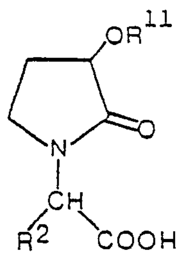

(III)

worin $R^2$ Wasserstoff oder ($C_1$-$C_7$)-Alkyl und $R^{11}$ Wasserstoff, ($C_1$-$C_8$)-Alkanoyl oder eine andere mittels Ammoniak leicht abspaltbare Gruppe bedeuten.

11. Verbindungen der allgemeinen Formel

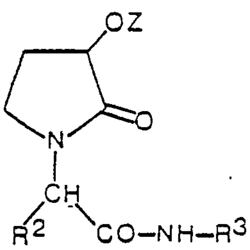

(IV)

worin $R^2$ und $R^3$ die in Anspruch 1 angegebene Bedeutung besitzen und Z eine Schutzgruppe bedeutet, wobei aber diese Schutzgruppe keine ($C_1$-$C_8$)-Alkanoylgruppe ist.

12. Verbindungen gemäss einem der Ansprüche 1 bis 9 als pharmazeutische Wirkstoffe.

13. Verbindungen gemäss einem der Ansprüche 1 bis 9 als der cerebralen Insuffizienz entgegenwirkende bzw. die intellektuelle Leistungsfähigkeit verbessernde Wirkstoffe.

14. Verfahren zur Herstellung von Verbindungen gemäss einem der Ansprüche 1 bis 9, dadurch gekennzeichnet, dass man

a) eine Verbindung der allgemeinen Formel

$$H_2N-R^3 \qquad II$$

worin $R^3$ die in Anspruch 1 angegebene Bedeutung besitzt, mit einer Carbonsäure der allgemeinen Formel

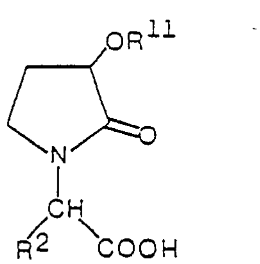

(III)

worin $R^2$ die in Anspruch 1 angegebene Bedeutung besitzt und $R^{11}$ Wasserstoff oder $(C_1\text{-}C_8)$-Alkanoyl oder — falls $R^3$ in Formel II Wasserstoff bedeutet — auch eine andere mittels Ammoniak abspaltbare Gruppe bedeutet, oder mit einem reaktionsfähigen funktionellen Derivat davon umsetzt, oder

b) eine Verbindung der allgemeinen Formel

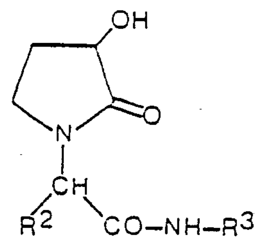

(Ib)

worin $R^2$ und $R^3$ die in Anspruch 1 angegebene Bedeutung besitzen, mit einem einen $(C_1\text{-}C_8)$-Alkanoylrest liefernden Mittel acyliert, oder

c) aus einer Verbindung der allgemeinen Formel

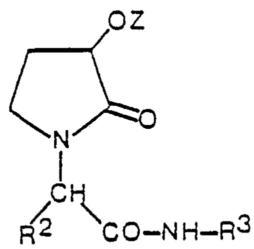

(IV)

worin $R^2$ und $R^3$ die in Anspruch 1 angegebene Bedeutung besitzen, und Z eine Schutzgruppe bedeutet, die mit Z bezeichnete Schutzgruppe abspaltet, und

d) erwünschtenfalls ein erhaltenes Gemisch von Diastereoisomeren in die entsprechenden Racemate auftrennt, und/oder

e) erwünschtenfalls ein erhaltenes Racemat einer Verbindung der allgemeinen Formel I, worin $R^1$ Wasserstoff und/oder $R^3$ einen basischen Rest bedeuten, in die optischen Antipoden spaltet, und/oder

f) erwünschtenfalls eine Verbindung der allgemeinen Formel I, welche basischen Charakter aufweist, in ein pharmazeutisch annehmbares Säureadditionssalz überführt.

15. Verfahren gemäss Anspruch 14, dadurch gekennzeichnet, dass $R^{11}$ Wasserstoff oder $(C_1\text{-}C_8)$-Alkanoyl bedeutet.

16. Arzneimittel, enthaltend eine Verbindung gemäss einem der Ansprüche 1 bis 9.

17. Der cerebralen Insuffizienz entgegenwirkendes bzw. die intellektuelle Leistungsfähigkeit verbesserndes Mittel, enthaltend eine Verbindung gemäss einem der Ansprüche 1 bis 9.

**Patentansprüche** (für den Vertragsstaat AT)

1. Verfahren zur Herstellung von Pyrrolidinderivaten der allgemeinen Formel

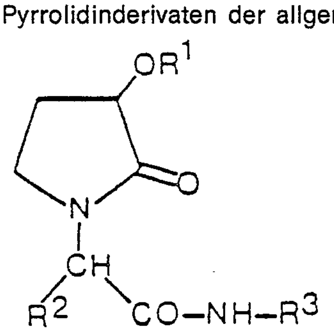

(I)

worin $R^1$ Wasserstoff oder $(C_1\text{-}C_8)$-Alkanoyl, $R^2$ Wasserstoff oder $(C_1\text{-}C_3)$-Alkyl und $R^3$ Wasserstoff, $(C_1\text{-}C_7)$-Alkyl oder einen Rest der Formel $-(CH_2)_n-NR^4R^5$, n eine ganze Zahl von 2 bis 4 und $R^4$ und $R^5$ je Wasserstoff oder $(C_1\text{-}C_7)$-Alkyl oder zusammen mit dem Stickstoffatom einen gegebenenfalls durch eine oder zwei $(C_1\text{-}C_7)$-Alkylgruppen substituierten Pyrrolidin-, Piperidin-, Piperazin- oder Morpholinrest bedeuten, und von Säureadditionssalzen von Verbindungen der Formel I, welche basischen Charakter aufweisen, dadurch gekennzeichnet, dass man

a) eine Verbindung der allgemeinen Formel

$$H_2N—R^3 \quad II$$

worin $R^3$ obige Bedeutung besitzt, mit einer Carbonsäure der allgemeinen Formel

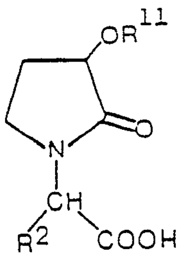

(III)

worin $R^2$ obige Bedeutung besitzt und $R^{11}$ Wasserstoff, oder ($C_1$-$C_8$)-Alkanoyl oder — falls $R^3$ in Formel II Wasserstoff bedeutet — auch eine andere mittels Ammoniak abspaltbare Gruppe bedeutet, oder mit einem reaktionsfähigen funktionellen Derivat davon umsetzt, oder

b) eine Verbindung der allgemeinen Formel

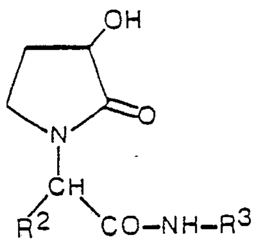

(Ib)

worin $R^2$ und $R^3$ obige Bedeutung besitzen, mit einem einen ($C_1$-$C_8$)-Alkanoylrest liefernden Mittel acyliert, oder

c) aus einer Verbindung der allgemeinen Formel

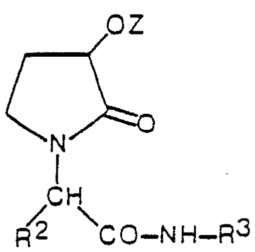

(IV)

worin $R^2$ und $R^3$ obige Bedeutung besitzen, und Z eine Schutzgruppe bedeutet, die mit Z bezeichnete Schutzgruppe abspaltet, und

d) erwünschtenfalls ein erhaltenes Gemisch von Diastereoisomeren in die entsprechenden Racemate auftrennt, und/oder

e) erwünschtenfalls ein erhaltenes Racemat einer Verbindung der allgemeinen Formel I, worin $R^1$ Wasserstoff und/oder $R^3$ einen basischen Rest bedeuten, in die optischen Antipoden spaltet, und/oder

f) erwünschtenfalls eine Verbindung der allgemeinen Formel I, welche basischen Charakter aufweist, in ein pharmazeutisch annehmbares Säureadditionssalz überführt.

2. Verfahren gemäss Anspruch 1, dadurch gekennzeichnet, dass $R^{11}$ Wasserstoff oder ($C_1$-$C_8$)-Alkanoyl bedeutet.

3. Verfahren gemäss Anspruch 1 oder 2, dadurch gekennzeichnet, dass $R^1$ Wasserstoff, $R^2$ Wasserstoff und $R^3$ Wasserstoff, 2-(Diisopropylamino)äthyl oder 2-(2,6-Dimethyl-1-piperidinyl)äthyl bedeuten.

4. Verfahren gemäss Anspruch 1 oder 2, dadurch gekennzeichnet, dass man (R/S)-cis-N-[2-(2,6-Dimethyl-1-piperidinyl)äthyl]-2-(3-hydroxy-2-oxo-1-pyrrolidinyl) acetamid herstellt.

5. Verfahren gemäss Anspruch 1 oder 2, dadurch gekennzeichnet, dass man (R/S)-2-(3-Hydroxy-2-oxo-1-pyrrolidinyl) acetamid herstellt.

6. Verfahren gemäss Anspruch 1 oder 2, dadurch gekennzeichnet, dass man (R)-cis-N-[2-(2,6-Dimethyl-1-piperidinyl) äthyl]-2-(3-hydroxy-2-oxo-1-pyrrolidinyl) acetamid herstellt.

7. Verfahren gemäss Anspruch 1 oder 2, dadurch gekennzeichnet, dass man (S)-cis-N-[2-(2,6-Dimethyl-1-piperidinyl) äthyl]-2-(3-hydroxy-2-oxo-1-pyrrolidinyl) acetamid herstellt.

8. Verfahren gemäss Anspruch 1 oder 2, dadurch gekennzeichnet, dass man (R)-2-(3-Hydroxy-2-oxo-1-pyrrolidinyl)-acetamid herstellt.

9. Verfahren gemäss Anspruch 1 oder 2, dadurch gekennzeichnet, dass man (S)-2-(3-Hydroxy-2-oxo-1-pyrrolidinyl-acetamid herstellt.

10. Verfahren gemäss Anspruch 1 oder 2, dadurch gekennzeichnet, dass man (R/S)-N-[2-(Diisopropylamino) äthyl]-2-(3-hydroxy-2-oxo-1-pyrrolidinyl) acetamid herstellt.

**Claims** (for the Contracting States : BE, CH, DE, FR, IT, LI, LU, NL, SE)

1. Pyrrolidine derivatives of the general formula

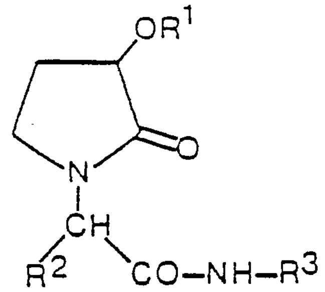

(I)

wherein $R^1$ signifies hydrogen or $(C_1\text{-}C_8)$-alkanoyl, $R^2$ signifies hydrogen or $(C_1\text{-}C_7)$-alkyl and $R^3$ signifies hydrogen, $(C_1\text{-}C_7)$-alkyl or a residue of the formula $-(CH_2)_n-NR^4R^5$, n signifies a whole number of 2 to 4 and $R^4$ and $R^5$ each signify hydrogen or $(C_1\text{-}C_7)$-alkyl or together with the nitrogen atom signify a pyrrolidine, piperidine, piperazine or morpholine residue which is optionally substituted by one or two $(C_1\text{-}C_7)$-alkyl groups, and acid addition salts of compounds of formula I which have basic character.

2. Compounds in accordance with claim 1, wherein $R^1$ signifies hydrogen, $R^2$ signifies hydrogen and $R^3$ signifies hydrogen, 2-(diisopropylamino) ethyl or 2-(2,6-dimethyl-1-piperidinyl) ethyl.

3. (R/S)-cis-N-[2,6-Dimethyl-1-piperidinyl) ethyl]-2-(3-hydroxy-2-oxo-1-pyrrolidinyl) acetamide.

4. (R/S)-2-(3-Hydroxy-2-oxo-1-pyrrolidinyl) acetamide.

5. (R)-cis-N-[2-(2,6-Dimethyl-1-piperidinyl) ethyl]-2-(3-hydroxy-2-oxo-1-pyrrolidinyl) acetamide.

6. (S)-cis-N-[2-(2,6-Dimethyl-1-piperidinyl) ethyl]-2-(3-hydroxy-2-oxo-1-pyrrolidinyl) acetamide.

7. (R)-2-(3-Hydroxy-2-oxo-1-pyrrolidinyl) acetamide.

8. (S)-2-(3-Hydroxy-2-oxo-1-pyrrolidinyl) acetamide.

9. (R/S)-N-[2-(Diisopropylamino) ethyl]-2-(3-hydroxy-2-oxo-1-pyrrolidinyl) acetamide.

10. Carboxylic acids of the general formula

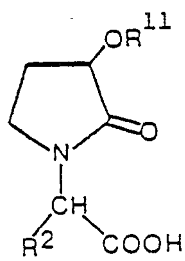

(III)

wherein $R^2$ signifies hydrogen or $(C_1\text{-}C_7)$-alkyl and $R^{11}$ signifies hydrogen, $(C_1\text{-}C_8)$-alkanoyl or another group which is readily cleavable by means of ammonia.

11. Compounds of the general formula

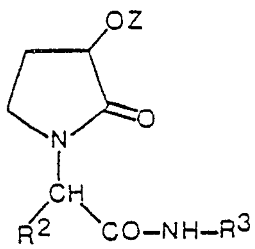

(IV)

wherein $R^2$ and $R^3$ have the significance given in claim 1 and Z signifies a protecting group, but whereby

this protecting group is not a ($C_1$-$C_8$)-alkanoyl group.

12. Compounds in accordance with any one of claims 1 to 9 as pharmaceutically active substances.

13. Compounds in accordance with any one of claims 1 to 9 as active substances counteracting cerebral insufficiency or improving intellectual capacity.

14. A process for the manufacture of compounds in accordance with any one of claims 1 to 9, characterized by

a) reacting a compound of the general formula

$$H_2N—R^3 \qquad II$$

wherein $R^3$ has the significance given in claim 1, with a carboxylic acid of the general formula

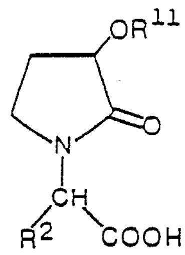

(III)

wherein $R^2$ has the significance given in claim 1 and $R^{11}$ signifies hydrogen or ($C_1$-$C_8$)-alkanoyl or — where $R^3$ in formula II signifies hydrogen — also another group which is cleavable by means of ammonia, or with a reactive functional derivative thereof, or

b) acylating a compound of the general formula

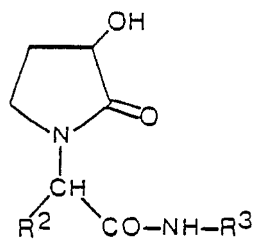

(Ib)

wherein $R^2$ and $R^3$ have the significance given in claim 1, with an agent yielding a ($C_1$-$C_8$)-alkanoyl residue, or

c) cleaving off the protecting group denoted by Z from a compound of the general formula

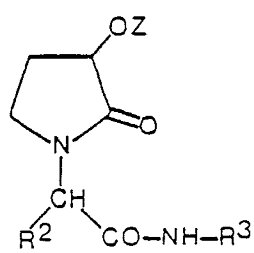

(IV)

wherein $R^2$ and $R^3$ have the significance given in claim 1 and Z signifies a protecting group, and

d) if desired, separating a mixture of diastereoisomers obtained into the corresponding racemates, and/or

e) if desired, resolving an obtained racemate of a compound of general formula I in which $R^1$ signifies hydrogen and/or $R^3$ signifies a basic residue into the optical antipodes, and/or

f) if desired, converting a compound of general formula I which has basic character into a pharmaceutically acceptable acid addition salt.

15. A process in accordance with claim 14, characterized in that $R^{11}$ signifies hydrogen or ($C_1$-$C_8$)-alkanoyl.

16. A medicament containing a compound in accordance with any one of claims 1 to 9.

17. A medicament counteracting cerebral insufficiency or improving intellectual capacity, containing a compound in accordance with any one of claims 1 to 9.

**Claims** (for the Contracting State AT)

1. A process for the manufacture of pyrrolidine derivatives of the general formula

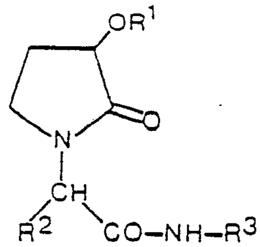

(I)

wherein $R^1$ signifies hydrogen or $(C_1\text{-}C_8)$-alkanoyl, $R^2$ signifies hydrogen or $(C_1\text{-}C_7)$-alkyl and $R^3$ signifies hydrogen, $(C_1\text{-}C_7)$-alkyl or a residue of the formula $-(CH_2)_n-NR^4R^5$, n signifies a whole number of 2 to 4 and $R^4$ and $R^5$ each signify hydrogen or $(C_1\text{-}C_7)$-alkyl or together with the nitrogen atom signify a pyrrolidine, piperidine, piperazine or morpholine residue which is optionally substituted by one or two $(C_1\text{-}C_7)$-alkyl groups, and acid addition salts of compounds of formula I which have basic character, characterized by

a) reacting a compound of the general formula

$$H_2N-R^3 \qquad II$$

wherein $R^3$ has the above significance, with a carboxylic acid of the general formula

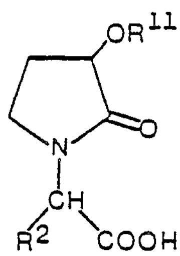

(III)

wherein $R^2$ has the above significance and $R^{11}$ signifies hydrogen or $(C_1\text{-}C_8)$-alkanoyl or — where $R^3$ in formula II signifies hydrogen — also another group which is cleavable by means of ammonia, or with a reactive functional derivative thereof, or

b) acylating a compound of the general formula

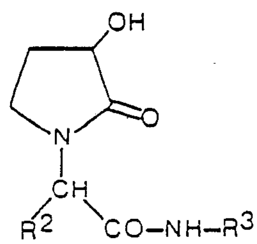

(Ib)

wherein $R^2$ and $R^3$ have the above significance, with an agent yielding a $(C_1\text{-}C_8)$-alkanoyl residue, or

c) cleaving off the protecting group denoted by Z from a compound of the general formula

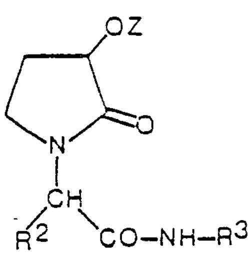

(IV)

wherein $R^2$ and $R^3$ have the above significance and Z signifies a protecting group, and

d) if desired, separating a mixture of diastereoisomers obtained into the corresponding racemates, and/or

e) if desired, resolving an obtained racemate of a compound of general formula I in which $R^1$ signifies hydrogen and/or $R^3$ signifies a basic residue into the optical antipodes, and/or

f) if desired, converting a compound of general formula I which has basic character into a pharmaceutically acceptable acid addition salt.

2. A process in accordance with claim 1, characterized in that $R^{11}$ signifies hydrogen or $(C_1-C_8)$-alkanoyl.

3. A process in accordance with claim 1 or 2, characterized in that $R^1$ signifies hydrogen, $R^2$ signifies hydrogen and $R^3$ signifies hydrogen, 2-(diisopropylamino) ethyl or 2-(2,6-dimethyl-1-piperidinyl) ethyl.

4. A process in accordance with claim 1 or 2, characterized in that (R/S)-cis-N-[2-(2,6-dimethyl-1-piperidinyl) ethyl]-2-(3-hydroxy-2-oxo-1-pyrrolidinyl)-acetamide is manufactured.

5. A process in accordance with claim 1 or 2, characterized in that (R/S)-2-(3-hydroxy-2-oxo-1-pyrrolidinyl) acetamide is manufactured.

6. A process in accordance with claim 1 or 2, characterized in that (R)-cis-N-[2-(2,6-dimethyl-1-piperidinyl) ethyl]-2-(3-hydroxy-2-oxo-1-pyrrolidinyl)-acetamide is manufactured.

7. A process in accordance with claim 1 or 2, characterized in that (S)-cis-N-[2-(2,6-dimethyl-1-piperidinyl) ethyl]-2-(3-hydroxy-2-oxo-1-pyrrolidinyl)-acetamide is manufactured.

8. A process in accordance with claim 1 or 2, characterized in that (R)-2-(3-hydroxy-2-oxo-1-pyrrolidinyl) acetamide is manufactured.

9. A process in accordance with claim 1 or 2, characterized in that (S)-2-(3-hydroxy-2-oxo-1-pyrrolidinyl) acetamide is manufactured.

10. A process in accordance with claim 1 or 2, characterized in that (R/S)-N-[2-(diisopropylamino) ethyl]-2-(3-hydroxy-2-oxo-1-pyrrolidinyl) acetamide is manufactured.

**Revendications** (pour les Etats contractants : BE, CH, DE, FR, IT, LI, LU, NL, SE)

1. Dérivés de la pyrrolidine de formule générale :

RESERVE 10 QG

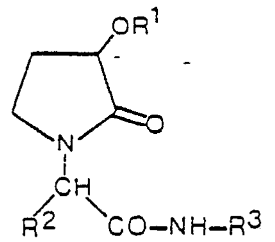

(I)

dans laquelle $R^1$ représente l'hydrogène ou un groupe alcanoyle en $C_1$-$C_8$, $R^2$ représente l'hydrogène ou un groupe alkyle en $C_1$-$C_7$ et $R^3$ représente l'hydrogène, un groupe alkyle en $C_1$-$C_7$ ou un groupe de formule $-(CH_2)_n-NR^4R^5$, n est un nombre entier de 2 à 4 et $R^4$ et $R^5$ représentent chacun l'hydrogène ou un groupe alkyle en $C_1$-$C_7$ ou forment ensemble et avec l'atome d'azote un reste pyrrolidine, pipéridine, pipérazine ou morpholine éventuellement substitué par un ou deux groupes alkyle en $C_1$-$C_7$, et les sels des composés de formule I présentant un caractère basique, formés par addition avec des acides.

2. Composés selon la revendication 1, dans lesquels $R^1$ représente l'hydrogène, $R^2$ l'hydrogène et $R^3$ l'hydrogène, un groupe 2-(diisopropylamino) éthyle ou 2-(2,6-diméthyl-1-pipéridinyl) éthyle.

3. Le (R/S)-cis-N-[2-(2,6-diméthyl-1-pipéridinyl)-éthyl]-2-(3-hydroxy-2-oxo-1-pyrrolidinyl) acétamide.

4. Le (R/S)-2-(3-hydroxy-2-oxo-1-pyrrolidinyl) acétamide.

5. Le (R)-cis-N-[2-(2,6-diméthyl-1-pipéridinyl) éthyl]-2-(3-hydroxy-2-oxo-1-pyrrolidinyl) acétamide.

6. Le (S)-cis-N-[2-(2,6-diméthyl-1-pipéridinyl) éthyl]-2-(3-hydroxy-2-oxo-1-pyrrolidinyl) acétamide.

7. Le (R)-2-(3-hydroxy-2-oxo-1-pyrrolidinyl) acétamide.

8. Le (S)-2-(3-hydroxy-2-oxo-1-pyrrolidinyl) acétamide.

9. le (R/S)-N-[2-(diisopropylamino) éthyl]-2-(3-hydroxy-2-oxo-1-pyrrolidinyl) acétamide.

10. Acides carboxyliques de formule générale :

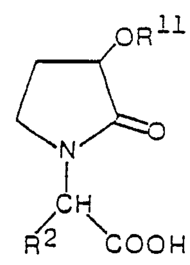

(III)

dans laquelle $R^2$ représente l'hydrogène ou un groupe alkyle en $C_1$-$C_7$ et $R^{11}$ représente l'hydrogène, un groupe alcanoyle en $C_1$-$C_8$ ou un autre groupe facile à éliminer par l'ammoniac.

11. Composés de formule générale :

RESERVE 10 QG

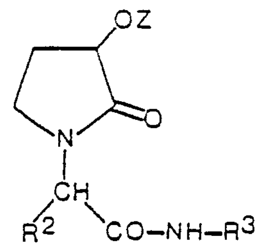

(IV)

dans laquelle $R^2$ et $R^3$ ont les significations indiquées dans la revendication 1, et Z représente un groupe protecteur qui, toutefois, ne peut consister en un groupe alcanoyle en $C_1$-$C_8$.

12. Composés selon l'une des revendications 1 à 9, en tant que substances actives pharmaceutiques.

13. Composés selon l'une des revendications 1 à 9, en tant que substances actives remédiant à l'insuffisance cérébrale ou améliorant la capacité intellectuelle.

14. Procédé de préparation de composés selon l'une des revendications 1 à 9, caractérisé en ce que :

a) on fait réagir un composé de formule générale :

$$H_2N{-}R^3 \qquad \text{II}$$

dans laquelle $R^3$ a les significations indiquées dans la revendication 1, avec un acide carboxylique de formule générale :

RESERVE 10 QG

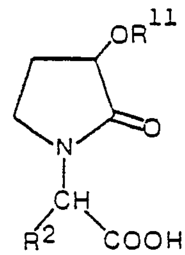

(III)

dans laquelle $R^2$ a les significations indiquées dans la revendication 1 et $R^{11}$ représente l'hydrogène ou un groupe alcanoyle en $C_1$-$C_8$ ou bien encore — lorsque $R^3$ de la formule II représente l'hydrogène — un autre groupe éliminable par l'ammoniac, ou avec un dérivé fonctionnel réactif d'un tel acide, ou bien

b) on acyle un composé de formule générale :

RESERVE 10 QG

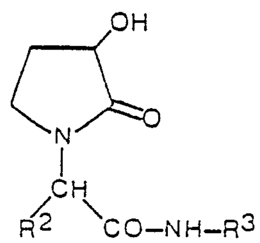

(Ib)

dans laquelle $R^2$ et $R^3$ ont les significations indiquées dans la revendication 1, par un agent apportant un groupe alcanoyle en $C_1$-$C_8$ ;

c) à partir d'un composé de formule générale :

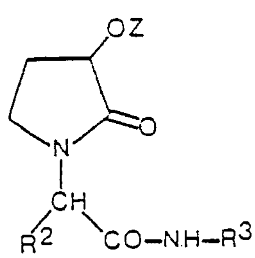

(IV)

dans laquelle $R^2$ et $R^3$ ont les significations indiquées dans la revendication 1, et Z représente un groupe protecteur, on élimine ce groupe protecteur Z, et

d) si on le désire, on sépare un mélange de diastéréoisomères ainsi obtenu en les racémates correspondants, et/ou

e) si on le désire, on résout un racémate d'un composé de formule générale I dans laquelle $R^1$ représente l'hydrogène et/ou $R^3$ un groupe basique, ainsi obtenu, en les antipodes optiques, et/ou

f) si on le désire, on convertit un composé de formule générale I présentant un caractère basique en un sel acceptable pour l'usage pharmaceutique par addition avec un acide.

15. Procédé selon la revendication 14, caractérisé en ce que $R^{11}$ représente l'hydrogène ou un groupe alcanoyle en $C_1$-$C_8$.

16. Médicament contenant un composé selon l'une des revendications 1 à 9.

17. Produit remédiant à l'insuffisance cérébrale et améliorant la capacité intellectuelle, contenant un composé selon l'une des revendications 1 à 9.

**Revendications** (pour l'Etat contractant AT)

1. Procédé de préparation de dérivés de la pyrrolidine de formule générale :

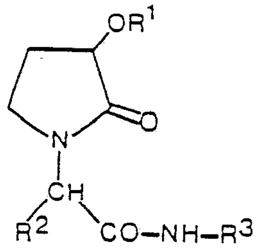

(I)

dans laquelle $R^1$ représente l'hydrogène ou un groupe alcanoyle en $C_1$-$C_8$, $R^2$ représente l'hydrogène ou un groupe alkyle en $C_1$-$C_7$ et $R^3$ représente l'hydrogène, un groupe alkyle en $C_1$-$C_7$ ou un groupe de formule $-(CH_2)_n-NR^4R^5$, n est un nombre entier de 2 à 4 et $R^4$ et $R^5$ représentent chacun l'hydrogène ou un groupe alkyle en $C_1$-$C_7$ ou forment ensemble et avec l'atome d'azote un groupe pyrrolidine, pipéridine, pipérazine ou morpholine éventuellement substitué par un ou deux groupes alkyle en $C_1$-$C_7$, et de sels de composés de formule I présentant un caractère basique formés par addition avec des acides, caractérisé en ce que :

a) on fait réagir un composé de formule générale :

$$H_2N-R^3 \qquad II$$

dans laquelle $R^3$ a les significations indiquées ci-dessus, avec un acide carboxylique de formule générale :

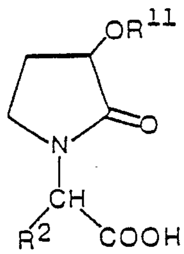

(III)

dans laquelle $R^2$ a les significations indiquées ci-dessus et $R^{11}$ représente l'hydrogène ou un groupe alcanoyle en $C_1$-$C_8$ ou bien encore — lorsque $R^3$ de la formule II représente l'hydrogène — un autre groupe éliminable par l'ammoniac, ou avec un dérivé fonctionnel réactif d'un tel acide, ou bien

b) on acyle un composé de formule générale :

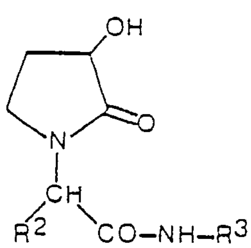

(Ib)

dans laquelle $R^2$ et $R^3$ ont les significations indiquées ci-dessus, par un réactif apportant un groupe alcanoyle en $C_1$-$C_8$, ou bien

c) à partir d'un composé de formule générale :

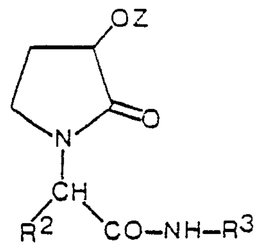

. (IV)

dans laquelle $R^2$ et $R^3$ ont les significations indiquées ci-dessus, et Z représente un groupe protecteur, on élimine ce groupe protecteur Z, et

d) si on le désire, on sépare un mélange de diastéréoisomères ainsi obtenu en les racémates correspondants, et/ou

e) si on le désire, on résout un racémate d'un composé de formule générale I, dans laquelle $R^1$ représente l'hydrogène et/ou $R^3$ un groupe basique, ainsi obtenu en les antipodes optiques, et/ou

f) si on le désire, on convertit un composé de formule générale I présentant un caractère basique en un sel acceptable pour l'usage pharmaceutique par addition avec un acide.

2. Procédé selon la revendication 1, caractérisée en ce que $R^{11}$ représente l'hydrogène ou un groupe alcanoyle en $C_1$-$C_8$.

3. Procédé selon la revendication 1 ou 2, caractérisé en ce que $R^1$ représente l'hydrogène, $R^2$ l'hydrogène et $R^3$ l'hydrogène, un groupe 2-(diisopropylamino) éthyle ou 2-(2,6-diméthyl-1-pipéridinyl) éthyle.

4. procédé selon la revendication 1 ou 2, caractérisé en ce que l'on prépare le (R/S)-cis-N-[2-(2,6-diméthyl-1-pipéridinyl) éthyl]-2-(3-hydroxy-2-oxo-1-pyrrolodinyl)-acétamide.

5. Procédé selon la revendication 1 ou 2, caractérisé en ce que l'on prépare le (R/S)-2-(3-hydroxy-2-oxo-1-pyrrolidinyl) acétamide.

6. Procédé selon la revendication 1 ou 2, caractérisé en ce que l'on prépare le (R)-cis-N-[2-(2,6-diméthyl-1-pipéridinyl) éthyl]-2-(3-hydroxy-2-oxo-1-pyrrolidinyl)-acétamide.

7. Procédé selon la revendication 1 ou 2, caractérisé en ce que l'on prépare le (S)-cis-N-[2-(2,6-diméthyl-1-pipéridinyl) éthyl]-2-(3-hydroxy-2-oxo-1-pyrrolidinyl)-acétamide.

8. Procédé selon la revendication 1 ou 2, caractérisé en ce que l'on prépare le (R)-2-(3-hydroxy-2-oxo-1-pyrrolidinyl) acétamide.

9. Procédé selon la revendication 1 ou 2, caractérisé en ce que l'on prépare le (S)-2-(3-hydroxy-2-oxo-1-pyrrolidinyl) acétamide.

10. Procédé selon la revendication 1 ou 2, caractérisé en ce que l'on prépare le (R/S)-N-[2-(diisopropylamino)-éthyl]-2-(3-hydroxy-2-oxo-1-pyrrolidinyl) acétamide.